# EUROPÄISCHE PATENTSCHRIFT

(11) **EP 1 893 620 B1**
(45) Veröffentlichungstag und Bekanntmachung des Hinweises auf die Patenterteilung: **14.09.2011**
(21) Anmeldenummer: 06742950.6
(22) Anmeldetag: 17.05.2006
(51) Int. Cl.: C07D 498/10, A61K 31/438, A61P 29/00

(54) **SUBSTITUIERTE SPIRO-VERBINDUNGEN UND DEREN VERWENDUNG ZUR HERSTELLUNG VON ARZNEIMITTELN GEGEN SCHMERZ**
SUBSTITUTED SPIRO COMPOUNDS AND THEIR USE FOR PRODUCING PAIN-RELIEF DRUGS
COMPOSES SPIRO SUBSTITUES, ET LEUR UTILISATION POUR PRODUIRE DES MEDICAMENTS CONTRE LA DOULEUR

(30) Priorität: 19.05.2005 DE 102005023784
(43) Veröffentlichungstag der Anmeldung: 05.03.2008
(60) Teilanmeldung: 10012043.5
(73) Patentinhaber: Grünenthal GmbH, 52078 Aachen (DE)
(72) Erfinder: SCHICK, Hans, D-13086 Berlin (DE); FRANK, Robert, 52070 Aachen (DE); REICH, Melanie, D-52072 Aachen (DE); JOSTOCK, Ruth, 52223 Stolberg (DE); BAHRENBERG, Gregor, D-52078 Aachen (DE); THEIL, Fritz, 12247 Berlin (DE); HENKEL, Birgitta, 12555 Berlin (DE)
(74) Vertreter: Bülle, Jan
(86) Internationale Anmeldenummer: PCT/EP2006/004651
(87) Internationale Veröffentlichungsnummer: WO 2006/122769

(56) Entgegenhaltungen:
- WO-A-97/33887
- WO-A-99/58139
- WO-A-03/000699
- SMALLHEER, JOANNE M. ET AL: "Synthesis and biological evaluation of nonpeptide integrin antagonists containing spirocyclic scaffolds" BIOORGANIC & MEDICINAL CHEMISTRY LETTERS , 14(2), 383-387 CODEN: BMCLE8; ISSN: 0960-894X, 2004, XP002390424

## Beschreibung

Die vorliegende Erfindung betrifft substituierte Spiro-Verbindungen, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz, insbesondere von neuropathischem Schmerz, stellt der Vanilloid-Rezeptor vom Subtyp-1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des-kardiovaskulären Systems; Störungen der Nahrungsaufnahme; Medikamentenabhängigkeit; Medikamentenmißbrauch und insbesondere Härninkontinenz.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass sich substituierte Spiro-Verbindungen der nachstehend angegebenen allgemeinen Formel I zur Bekämpfung von Schmerz eignen und eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanillold-Rezeptoren 1 (VR1/TRPV1) vermittelt werden.

Ein Gegenstand der vorliegenden Erfindung sind daher substituierte Spiro-Verbindungen der allgemeinen Formel I, 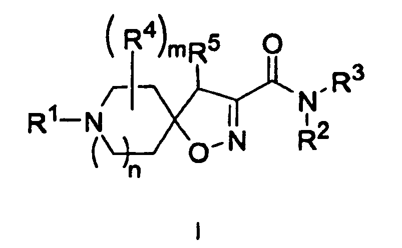 worin
- m: gleich 0, 1, 2, 3 oder 4 ist,
- n: gleich 0 ist,
- R¹: für einen linearen oder verzweigten, gesättigten oder ungesättigten, ggf. substituierten C₁₋₁₀-aliphatischen Rest;
für einen ungesättigten oder gesättigten, ggf. substituierten 3-, 4-, 5-, 6-, 7-, 8-, oder 9-gliedrigen cycloaliphatischen Rest; der mit einem gesättigten, ungesättigten oder aromatischen, ggf. substituierten mono- oder polyzyklischen Ringsystem kondensiert sein kann,
für einen ggf. substituierten 5- bis 14-gliedrigen Aryl- oder Heteroaryt-Rest, der mit einem gesättigten oder ungesättigten, ggf. substituierten mono- oder polyzyklischen Ringsystem kondensiert sein kann,
oder für eine -C(=S)-NR⁸R⁹-Gruppe steht;
für -(CHR²⁶)-X_{q}-(CHR²⁷)ᵣ-Yₛ-(CHR²⁸)ₜ-Zᵤ-R²⁹ mit q = 0 oder 1, r = 0 oder 1, s = 0 oder 1, t = 0 oder 1, u = 0 oder 1, worin X, Y und Z, unabhängig voneinander, jeweils für 0, S, NH, N(CH₃), N(C₂H₅) oder N[CH(CH₃)₂] stehen, oder
sofern m ungleich 0 und/oder n ungleich 1 und/oder R⁵ ungleich H ist,
zusätzlich für eine -C(=O)-NR⁶R⁷-Gruppe stehen kann,
- R²: für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, steht;
- R³: für einen Wasserstoff Rest;
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, steht;
oder
- R² und R³: zusammen mit dem sie verbindenden Stickstoffatom als Ringglied einen gesättigten oder ungesättigten, ggf. substituierten 4-, 5-, 6-, 7-, 8- oder 9- gliedrigen heterocycloaliphatischen Rest bilden, der mit einem gesättigten, ungesättigten oder aromatischen, ggf. substituierten mono- oder polyzyklischen Ringsystem kondensiert sein kann,
- R⁴: für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R¹⁰-Gruppe, für eine -NR¹¹R¹²-Gruppe, für eine -C(=O)-R¹³-Gruppe, für eine -C(=O)-O-R¹⁴-Gruppe, für eine -O-C(=O)- R¹⁵-Gruppe, für eine -NH-C(=O)-R¹⁶-Gruppe, für eine -NR¹⁷-C(=O)-R¹⁸- Gruppe, für eine -C(=O)-NH₂-Gruppe, für eine -C(=O)-NH-R¹⁹-Gruppe, für eine -C(=O)-NR²⁰R²¹-Gruppe, für eine -O-R²²-Gruppe, für eine -S-R²³-Gruppe, oder für einen linearen oder verzweigten, gesättigten oder ungesättigten, ggf. substituierten C₁₋₁₀ aliphatischen Rest steht;
- R⁵: für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH- C(=O)-H-Gruppe, für eine Oxo-Gruppe (=O), für eine -NH-R¹⁰-Gruppe, für eine -NR¹¹R¹²-Gruppe, für eine -C(=O)-R¹³-Gruppe, für eine -C(=O)-O-R¹⁴-Gruppe, für eine -O-C(=O)-R¹⁵-Gruppe, für eine -NH-C(=O)-R¹⁸-Gruppe, für eine -NR¹⁷- C(=0)-R¹⁸-Gruppe, für eine -C(=O)-NH₂-Gruppe, für eine -C(=O)-NH-R¹⁹- Gruppe, für eine -C(=O)-NR²⁰R²¹-Gruppe, für eine -O-R²²-Gruppe, für eine -S- R²³-Gruppe, für einen linearen oder verzweigten, gesättigten oder ungesättigten, ggf. substituierten C₁₋₁₀ aliphatischen Rest steht;
für einen ggf. substituierten 5- bis 14-gliedrigen Aryl- oder Heteroaryl-Rest
oder für -(CH₂)ₐₐ-R³⁴ mit aa = 1, 2, 3 oder 4 steht;
- R⁶ und R⁸,: unabhängig voneinander, jeweils
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryt-Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH₂)ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-Zₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
- R⁷ und R⁹,: unabhängig voneinander, jeweils für
für einen Wasserstoff-Rest,
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)-C(=O)-R²⁴ mit bb = 0 oder 1 und cc = 0 oder 1;
für -(CHR³⁶)-(CH₂)_{dd}-(CH₂)ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-4ₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
und
- R¹⁰ bis R²⁵,: unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatlschen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
oder für -(CH₂)ₘₘ-R⁴² mit mm gleich 1, 2 oder 3 stehen;
- R²⁶, R²⁷, R²⁸, R³⁰, R³¹, R³², R³⁵,:
- R³⁶, R³⁷, R³⁸, R³⁹ und R⁴⁰,: unabhängig voneinander, jeweils
für einen Wasserstoff Rest;
für einen linearen oder verzweigten, gesättigten oder ungesättigten, ggf. substituierten C₁₋₁₀ aliphatischen Rest,
oder für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann, stehen;
- R²⁹, R³³ und R⁴¹,: unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit 1, 2, 3, 4 oder 5 linearen oder verzweigten, ggf. substituierten C₁₋₅-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann, stehen;
und
- R³⁴ und R⁴²,: unabhängig voneinander, jeweils
für einen ggf. substituierten 5- bis 14-gliedrigen Aryl- oder Heteroaryl-Rest stehen;
wobei
die vorstehend genannten C₁₋₁₀ aliphatischen Reste und C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -SH und - NH₂ substituiert sein können;
die vorstehend genannten cycloaliphatischen Reste und heterocycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, - CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, - (CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -0-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten heterocycloaliphatischen Reste jeweils ggf. 1, 2 oder 3 zusätzliche(s) Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃,-S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅₋Alkyl)₂, -S(=O)₂-C₁₋₅-Alkyl, - S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt können die Substituenten der vorstehend genannten aliphatischen Reste in Position der Substituenten R¹ bis R²⁵ unabhängig voneinander aus der Gruppe bestehend aus F, Cl, Br, I, -CN, -NO₂, -OH, -SH und -NH₂ ausgewählt werden.

Bevorzugt können die Reste R⁵, R² und R³ Alkylen-, Alkenylen- oder Alkinylen-Gruppen aufweisen, die jeweils mit Substituenten unabhängig voneinander ausgewählt aus der Gruppe bestehend aus F, Cl, Br, -OH, -SH, -NH₂, -CN, -NO₂ und Phenyl substituiert sein können; wobei der Phenyl-Rest mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H_{5,} -C(=O)-O-C(CH₃)₃, -O-C(=O)-CH₃, -O-C(=O)-C₂H₅, -0-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann.

Bevorzugt bilden dir Reste R² und R³ zusammen mit dem sie verbindenden Stickstoffatom einen heterocycloaliphatischen Rest, der mit Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -NH-C₁₋₅-Alkyl, N(C₁₋₅₋Alkyl)₂, -NH-C(=O)-O-C₁₋₅-Alkyl, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -0-CF₃, -S-CF₃, Phenyl und -O-Benzyl substituiert sein kann.

Aliphatische Reste umfassen im Sinne dieser Erfindung azyklische gesättigte oder ungesättigte Kohlenwasserstoffreste, die verzweigt oder geradkettig sowie unsubstituiert oder einfach substituiert oder mehrfach, beispielsweise 1-, 2-, 3-, 4-, 5-, 6-, 7-, 8- oder 9-fach, gleich oder verschieden substituiert sein können, mit 1 bis 20 (d.h. 1, 2, 3, 4, 5, 6, 7, 8, 9, 10, 11, 12, 13, 14, 15, 16, 17, 18, 19 oder 20), bevorzugt 1 bis 12 (d.h. 1, 2, 3, 4, 5, 6, 7, 8, 9, 10, 11 oder 12), besonders bevorzugt 1 bis 6 (d.h. 1, 2, 3, 4, 5 oder 6) Kohlenstoffatomen, d.h. C₁₋₂₀-, C₁₋₁₂-, C₁₋₆-Alkyle, C₂₋₂₀-, C₂₋₁₂-, C₂₋₆-Alkenyle und C₂₋₂₀-, C₂₋₁₂-, C₂₋₆-Alkinyle. Dabei weisen Alkenyle mindestens eine C-C-Doppelbindung und Alkinyle mindestens eine C-C-Dreifachbindung auf. Beispielhaft seien aliphatische Reste ausgewählt aus der Gruppe bestehend aus Methyl, Ethyl, n-Propyl, Isopropyl, n-Butyl, Isobutyl, sec-Butyl, tert-Butyl, n-Pentyl, Isopentyl, neo-Pentyl, n-Hexyl, 2-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Ethenyl (Vinyl), Ethinyl, Propenyl (-CH₂CH=CH₂, -CH=CH-CH₃, -C(=CH₂)-CH₃), 2-Methyl-propenyl, Propinyl (-CH₂-C≡CH, -C≡C-CH₃), Butenyl, Butinyl, Pentenyl, Pentinyl, Hexenyl, Hexinyl, Octenyl und Octinyl genannt.

Die vorstehend genannten aliphatischen Reste können bevorzugt 1, 2 oder 3 Heteroatome unabhängig voneinander ausgewählt aus der Gruppe umfassend Sauerstoff, Schwefel und Stickstoff, einschließlich -N(H)- und -N(C₁₋₆-Alkyl), als Kettenglieder aufweisen.

Beispielhaft für aliphatische Reste, die 1, 2 oder 3 Heteroatome aufweisen, seien -(CH₂)-(CH₂)-O-CH₃, -(CH₂)-(CH₂)-(CH₂)-O-CH₃, -(CH₂)-(CH₂)-(CH₂)-N(C₂H₅)-(C₂H₅), -(CH₂)-(CH₂)-S-CH₃, -(CH₂)-(CH₂)-(CH₂)-S-CH₃, -(CH₂)-(CH₂)-(CH₂)-N(CH₃)-(CH₃) und -(CH₂)-O-CH₃ genannt.

Im Zusammenhang mit aliphatischen Resten versteht man unter dem Begriff "substituiert" - soweit nicht anders definiert - im Sinne der vorliegenden Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch beispielsweise F, Cl, Br, I, -CN, -NO₂, -OH, -SH und -NH₂, wobei die mehrfache Substitution entweder an verschiedenen oder an gleichen Atomen mehrfach, z.B. zwei- oder dreifach, erfolgt, beispielsweise dreifach am gleichen C-Atom wie im Falle von -CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Bevorzugte substituierte aliphatische Reste sind -CH₂-Cl, -CH₂-Br, -CH₂-CH₂-Cl, -CH₂-CH₂-Br, -CH₂-CH₂-CH₂-Br und -CH₂-CH₂-CH₂-Cl.

Cycloaliphatische Reste im Sinne der vorliegenden Erfindung sind zyklische gesättigte oder ungesättigte Kohlenwasserstoff-Reste mit 3, 4, 5, 6, 7, 8, 9, 10, 11, 12, 13, 14, 15 oder 16, bevorzugt 3, 4, 5, 6, 7 oder 8 Kohlenstoffatomen, wobei jeder Rest unsubstituiert oder einfach oder mehrfach, beispielsweise 1-, 2-, 3-, 4-, 5-, 6-, 7-, 8- oder 9-fach, gleich oder verschieden substituiert sein kann. Cycloaliphatische Reste können bevorzugt 1, 2, 3, 4 oder 5 Heteroatome unabhängig voneinander ausgewählt aus der Gruppe bestehend aus Sauerstoff, Stickstoff (NH) und Schwefel als Ringglieder aufweisen.

Beispielhaft für cycloaliphatische Reste, die ggf. mit 1 oder 2 linearen oder verzweigten C₁₋₅-Alkylen-Gruppen überbrückt und mit einen mono- oder polyzyklischen Ringsystem kondensiert sein können, seien Cyclopropyl, Cyclobutyl, Cyclopentyl, Cyclohexyl, Cyclooctyl, Cyclononyl, Cyclodecyl, Cycloundecyl, Cyclododecyl, Cycloheptyl, Cyclopentenyl, Cyclohexenyl, Cycloheptenyl, [6,6]-Dimethyl-[3.1.1]-bicycloheptyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl, (1,2,3,4)-Tetrahydrochinazolinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl und (1,3)-Thiazolidinyl genannt.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien [1,3]-Benzodioxotyl, [1,4]-Benzodioxanyl, [1,2,3,4]-Tetrahydronaphthyl, [1,2,3,4]-Tetrahydrochinolinyl, [1,2,3,4]-Tetrahydrochinazolinyl und [3,4]-Dihydro-2H-1,4-benzoxazinyl genannt.

Im Zusammenhang mit cycloaliphatischen Resten und mono- oder polyzyklischen Ringsystemen versteht man unter dem Begriff "substituiert" - soweit nicht anders definiert - im Sinne dieser Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch beispielsweise Oxo (=O), Thioxo (=S), F, Cl, Br, I, -CN, -CF₃, -SF₅, -OH, -O-C₁₋₅-Alkyl, -NH₂, -NO₂,-O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H,-C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzylsubstituiert sein kann. Die mehrfache Substitution kann entweder an verschiedenen oder an gleichen Atomen mehrfach, z.B. zwei- oder dreifach, erfolgen. Die Mehrfachsubstitution kann mit dem gleichen oder mit verschiedenen Substituenten erfolgen.

Unter dem Ausdruck Aryl-Rest ist für die Zwecke der vorliegenden Erfindung bevorzugt ein Rest zu verstehen, der aus der Gruppe, die Phenyl, Naphthyl, Phenanthrenyl und Anthracenyl umfasst, ausgewählt ist und unsubstituiert oder einfach oder mehrfach gleich oder verschieden substituiert ist. Bevorzugt ist Aryl ein unsubstituiertes oder einfach substituiertes oder mehrfach, d. h. zwei-, drei- vier oder fünffach, gleich oder verschieden substituiertes Phenyl, 1-Naphthyl oder 2-Naphthyl.

Heteroaryl-Reste im Sinne der vorliegenden Erfindung sind solche Heterozyklen, die heteroaromatisch sind. Heteroaryl-Reste sind bevorzugt 5- bis 14-gliedrig, d. h. 5-, 6-, 7-, 8-, 9-, 10-, 11-, 12-, 13- oder 14-gliedrig und weisen bevorzugt 1, 2, 3, 4 oder 5 Heteroatome unabhängig voneinander ausgewählt aus der Gruppe umfassend Sauerstoff, Stickstoff und Schwefel auf. Jeder Heteroaryl-Rest kann unsubstituiert oder einfach substituiert oder mehrfach, d. h. zwei-, drei-, vier- oder fünffach, gleich oder verschieden substituiert vorliegen.

Beispielhaft für Heteroaryl-Reste im Sinne der vorliegenden Erfindung seien Thiophenyl, Furanyl, Pyrrolyl, Pyrazolyl, Pyranyl, Triazolyl, Pyridiriyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl, Benzothiazolyl, Benzo[2,1,3]thiadiazolyl, [1,2,3]-Benzothiadiazolyl, [2,1,3]-Benzoxadiazolyl und [1,2,3]-Benzoxadiazolyl genannt.

In Bezug auf Aryl- und Heteroaryl-Reste versteht man im Sinne dieser Erfindung unter "substituiert" die ein- oder mehrfache, z.B. zwei-, drei-, vier- oder fünffache, Substitution eines oder mehrerer Wasserstoffatome des Ringsystems durch geeignete Substituenten. Soweit die Bedeutung dieser geeigneten Substituenten im Zusammenhang mit Aryl- oder Heteroaryl-Resten nicht an anderer Stelle der Beschreibung oder in den Ansprüchen definiert ist, sind geeignete Substituenten F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Pyridazinyl, - S(=O)₂-Phenyl, -O-Phenyl, -O-benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -0-CF₃, -S-CF₃, Phenyl und -O-Benzyl substituiert sein kann.
Die Mehrfachsubstitution erfolgt dabei mit dem gleichen oder mit unterschiedlichen Substituenten.

Die vorstehend genannten linearen oder verzweigten Alkylen-, Alkenylen- oder Alkinylen-Gruppen weisen bevorzugt 1 bis 5 Kohlenstoffatome auf, d.h. es handelt sich um C₁₋₅-Alkylen, C₂₋₅-Alkenylen oder C₂₋₅-Alkinylen-Gruppen, die jeweils unsubstituiert oder mit 1, 2, 3, 4 oder 5 Substituenten unabhängig voneinander ausgewählt aus der Gruppe bestehend aus F, Cl, Br, -OH, -SH, -NH₂, -CN, NO₂ und Phenyl substituiert sein können.

Die vorstehend genannten Alkylen-, Alkenylen- oder Alkinylen-Gruppen weisen ggf. jeweils 1 oder 2 Heteroatom(e) ausgewählt aus der Gruppe bestehend aus Sauerstoff, Stickstoff, d. h. -N(H)- und -N(C₁₋₆-Alkyl)-, und Schwefel als Kettenglied(er) auf.

Bevorzugt können Alkylen-Gruppen ausgewählt werden aus der Gruppe bestehend aus -(CH₂)-, -(-CH₂)₂-, C(H)(CH₃)-, -C(CH₃)₂-, -(CH₂)₃-, -(CH₂)₄-, -(CH₂)₅-, -C(H)(CH₃)-(CH₂)-, -C(H)(C₂H₅)-(CH₂)-, -C(Phenyl)₂-, -C(H)(Phenyl)-, -(CH₂)-O-, -(CH₂)-N(CH₃)-, -(CH₂)-S-, -(CH₂)-(CH₂)-N(CH₃)- und -(CH₂)-(CH₂)-N(C₂H₅)-.

Der Fachmann versteht, dass sich für m gleich 0 die folgende allgemeine Formel Ic ergibt:

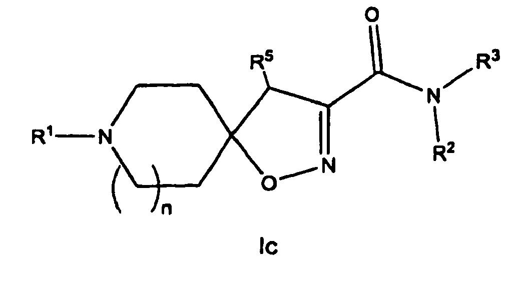

Ferner können erfindungsgemäße substituierte Spiro-Verbindungen der allgemeinen Formel I bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM zeigen.

Dabei wird im FLIPR-Assay der Ca ²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von erfindungsgemäßen Verbindungen der vorstehend angegebenen allgemeinen Formel I gemäß dem wenigstens eine Verbindung der allgemeinen Formel II, 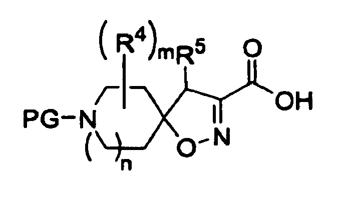 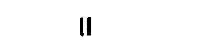 worin R⁴, R⁵, m und n die vorstehend genannte Bedeutung haben und PG für eine Schutzgruppe, bevorzugt für eine Benzyloxycarbonyl- oder tert-Butyloxycarbonyl-Gruppe, steht, in einem Reaktionsmedium in Gegenwart wenigstens eines Kupplungsreagenzes ggf. in Gegenwart wenigstens einer Base mit einer Verbindung der allgemeinen Formel HNR²R³, worin R² und R³ die vorstehend genannte Bedeutung haben, zu wenigstens einer Verbindung der allgemeinen Formel III, 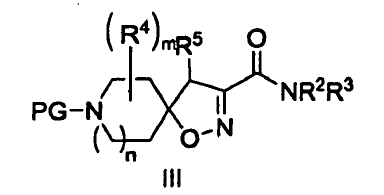 worin R², R³, R⁴, R⁵, m, n und PG die vorstehend genannte Bedeutung haben, umgesetzt wird und diese ggf. gereinigt und/oder isoliert wird
und
wenigstens eine Verbindung der allgemeinen Formel III in einem Reaktionsmedium in Gegenwart wenigstens einer Säure, vorzugsweise in Gegenwart wenigstens einer anorganischen oder organischen Säure ausgewählt aus der Gruppe bestehend aus Salzsäure, Schwefelsäure, Essigsäure und Trifluoressigsäure, oder in Gegenwart von Wasserstoff und eines Katalysators, vorzugsweise eines Katalysators basierend auf Palladium oder Platin, zu wenigstens einer Verbindung der allgemeinen Formel IV, 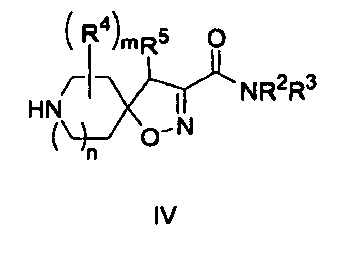 worin R², R³, R⁴, R⁵, m und n die vorstehend genannte Bedeutung haben, umgesetzt wird und diese ggf. gereinigt und/oder isoliert wird
und
wenigstens eine Verbindung der allgemeinen Formel IV in einem Reaktionsmedium mit wenigstens einem Isocyanat der allgemeinen Formel F⁶-N=C=O, worin R⁶ die vorstehend genannte Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R², R³, R⁴, R⁵, m und n die vorstehend genannte Bedeutung haben und R¹ für -C(=O)-NR⁶R⁷ steht, wobei R⁶ die vorstehend genannte Bedeutung hat und R⁷ für einen Wasserstoff-Rest steht, und diese ggf. gereinigt und/oder isoliert wird
oder
wenigstens eine Verbindung der allgemeinen Formel IV in einem Reaktionsmedium mit wenigstens einem Isothiocyanat der allgemeinen Formel S=C=N-R⁸, worin R⁸ die vorstehend genannte Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R², R³, R⁴, R⁵, m und n die vorstehend genannte Bedeutung haben und R¹ für -C(=S)-N-R⁸R⁹ steht, wobei R⁸ die vorstehend genannte Bedeutung hat und R⁹ für einen Wasserstoff-Rest steht, und diese ggf. gereinigt und/oder isoliert wird
und ggf. wenigstens eine Verbindung der allgemeinen Formel I, worin R², R³, R⁴, R⁵, m und n die vorstehend genannte Bedeutung haben und R¹ für -C(=O)-NR⁶R⁷ oder - C(=S)-N-R⁶R⁹ steht, worin R⁷ und R⁹ jeweils für einen Wasserstoff-Rest stehen, in einem Reaktionsmedium, in Gegenwart wenigstens einer Base, bevorzugt in Gegenwart wenigstens eines Metalihydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kallum-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁷ oder der allgemeinen Formel LG-R⁹, worin LG für eine Abgangsgruppe, bevorzugt für ein Halogen-Atom, besonders bevorzugt für ein Chloratom steht, und R⁷ und R⁹ die vorstehend genannte Bedeutung mit Ausnahme von Wasserstoff haben, zu wenigstens einer Verbindung der allgemeinen Formel **I** umgesetzt wird, worin R¹ bis R⁵, m und n die vorstehend genannte Bedeutung haben, und diese ggf. gereinigt und/oder isoliert wird,
oder
wenigstens eine Verbindung der allgemeinen Formel IV in einem Reaktionsmedium, in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-R¹, worin R¹ die vorstehend genannte Bedeutung hat, mit Ausnahme von -C(=O)-NR⁶R⁷ und - C(=S)-NR⁸R⁹, und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ bis R⁵, m und n die vorstehend genannte Bedeutung haben, und diese ggf. gereinigt und/oder isoliert wird
oder
wenigstens eine Verbindung der allgemeinen Formel IV in einem Reaktionsmedium, in Gegenwart wenigstens eines Reduktionsmittels, mit wenigstens einer Verbindung der allgemeinen Formel R¹-C(=O)-H, worin R¹ die vorstehend genannte Bedeutung hat, mit Ausnahme von -C(=O)-NR⁶R⁷ und -C(=S)-NR⁸R⁹, zu wenigstens einer Verbindung der allgemeinen Formel I umgesetzt wird, worin R¹ bis R⁵, m und n die vorstehend genannte Bedeutung haben, und diese ggf. gereinigt und/oder isoliert wird.

Die erfindungsgemäßen Verfahren zur Herstellung substituierter Spiro-Verbindungen der vorstehend angegebenen allgemeinen Formel I sind auch in den nachfolgenden Schemata 1 und 2 wiedergegeben.

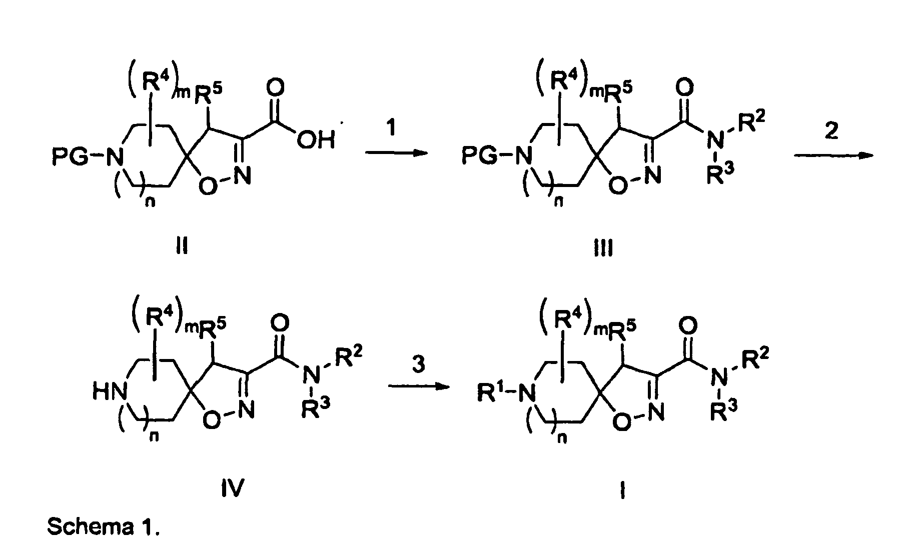

In Stufe 1 werden Verbindungen der vorstehend angegebenen allgemeinen Formel II mit Aminen der allgemeinen Formel HNR²R³, worin R¹ und R² die vorstehend genannte Bedeutung haben, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluroniom tetrafluoroborat (TBTU), 1-Hydroxybenzotriazol (HOBt) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, N-Methylmorpholin, Pyridin, N,N-Dimethylaminopyridin und Diisopropylethylamin vorzugsweise bei Temperaturen von -70°C bis 100°C zu Verbindungen der allgemeinen Formel I umgesetzt.

In Stufe 2. werden Verbindungen der allgemeinen Formel III, worin PG für eine tert-Butyloxycarbonyl-Gruppe steht, in einem Reaktionsmedium vorzugsweise ausgewählt aus der Gruppe bestehend aus Methanol, Ethanol, Isopropanol, Wasser, Diethylether, Tetrahydrofuran sowie entsprechenden Mischungen in Gegenwart wenigstens einer Säure vorzugsweise ausgewählt aus der Gruppe bestehend aus Salzsäure, Schwefelsäure, Trifluoressigsäure und Essigsäure bei Temperaturen von vorzugsweise 20 bis 30 °C zu Verbindungen der allgemeinen Formel IV umgesetzt. Besonders bevorzugt erfolgt die Umsetzung der Verbindung der allgemeinen Formel III in einer 5 M Salzsäure-Lösung in Isopropanol bei einer Temperatur von vorzugsweise 20 bis 30 °C zu einer Verbindung der allgemeinen Formel IV in Form eines entsprechenden Hydrochlorids.

Alternativ werden Verbindungen der allgemeinen Formel III, worin PG für eine Benzyloxycarbonyl-Gruppe steht, in einem Reaktionsmedium vorzugsweise ausgewählt aus der Gruppe bestehend aus Diethylether, Tetrahydrofuran, Dioxan, Acetonitril, Toluol und entsprechenden Mischungen in Gegenwart von Wasserstoff und Palladium auf Kohle bei einer Temperatur von vorzugsweise 20 bis 80 °C zu einer Verbindung der allgemeinen Formel IV umgesetzt.

Sofern Verbindungen der allgemeinen Formel IV in Form eines entsprechenden Hydrochlorids vorliegen, werden diese in einem Reaktionsmedium, vorzugsweise ausgewählt aus der Gruppe bestehend aus Dioxan, Tetrahydrofuran, Diethylether, Methanol, Ethanol, Isopropanol, Wasser und entsprechenden Mischungen, in Gegenwart einer anorganischen Base, vorzugsweise unter Zusatz eines Metallhydroxids, beispielsweise Natriumhydroxid, Kaliumhydroxid oder Lithiumhydroxid, bei Temperaturen von vorzugsweise 0°C bis 30°C in die entsprechende Basen der allgemeinen Formel IV umgesetzt.

In Stufe 3 werden Verbindungen der allgemeinen Formel IV mit einem Isocyanat der allgemeinen Formel R⁶-N=C=O, worin R⁶ die vorstehend angegebene Bedeutung hat, oder mit einem Isothiocyanat der allgemeinen Formel R⁸-N=C=S, worin R⁸ die vorstehend angegebene Bedeutung hat, in einem Reaktionsmedium, vorzugsweise ausgewählt aus der Gruppe bestehend aus Acetonitril, Toluol, Dimethylformamid, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, N-Methylmorpholin, Pyridin, 4,4-Dimethylaminopyridin und Diisopropylethylamin, zu Verbindungen der allgemeinen Formel I umgesetzt, worin R¹ für -C(=O)-NR⁸R⁷ oder -C(=S)-NR⁸R⁹ steht, und R⁷ und R⁹ jeweils für einen Wasserstoff-Rest stehen.

Ebenfalls werden in Stufe 3. Verbindungen der allgemeinen Formel IV mit Verbindungen der allgemeinen Formel LG-R¹, worin R¹ die vorstehend angegebene Bedeutung hat und LG für eine Abgangsgruppe, bevorzugt für ein Halogen-Atom, besonders bevorzugt für ein Chloratom steht, in einem Reaktionsmedium, vorzugsweise ausgewählt aus der Gruppe bestehend aus Dichlormethan, Toluol, Tetrahydrofuran, Acetonitril, Diethylether, Dioxan und entsprechenden Mischungen ggf. in Gegenwart wenigstens einer Base, bevorzugt in Gegenwart wenigstens eines Metallhydridsalzes, besonders bevorzugt in Gegenwart von Natrium- und/oder Kaliumhydrid, zu Verbindungen der allgemeinen Formel I, worin R¹ die vorstehend angegebene Bedeutung mit Ausnahme von -C(=O)-NR⁶R⁷ und -C(=S)-NR⁸R⁹ hat, umgesetzt.

Alternativ werden in Stufe 1. Verbindungen der allgemeinen Formel IV mit Verbindungen der allgemeinen Formel R¹-C(=O)-H, worin R¹ die vorstehend genannte Bedeutung hat, in einem Reaktionsmedium, vorzugsweise ausgewählt aus der Gruppe bestehend aus Diethylether, Tetrahydrofuran, Methanol, Ethanol, Dichlormethan, Toluol und entsprechenden Mischungen, unter Zusatz wenigstens eines Reduktionsmittels, vorzugsweise unter Zusatz wenigstens eines Reduktionsmittels ausgewählt aus der Gruppe bestehend aus Natriumborhydrid, Natriacetoxyborhydrid, Natriumcyanoborhydrid und Boran-Pyridin-Komplex (Pyridin-Boran, BH₃•C₅H₅N), besonders bevorzugt in Gegenwart von Boran-Pyridin-Komplex, zu Verbindungen der allgemeinen Formel I, worin R¹ die vorstehend angegebene Bedeutung mit Ausnahme von -C(=O)-NR⁶R⁷ und -C(=S)-NR⁸R⁹ hat, umgesetzt.

Die Verbindungen der allgemeinen Formel II lassen sich wie in Schema 2. beschrieben erhalten.

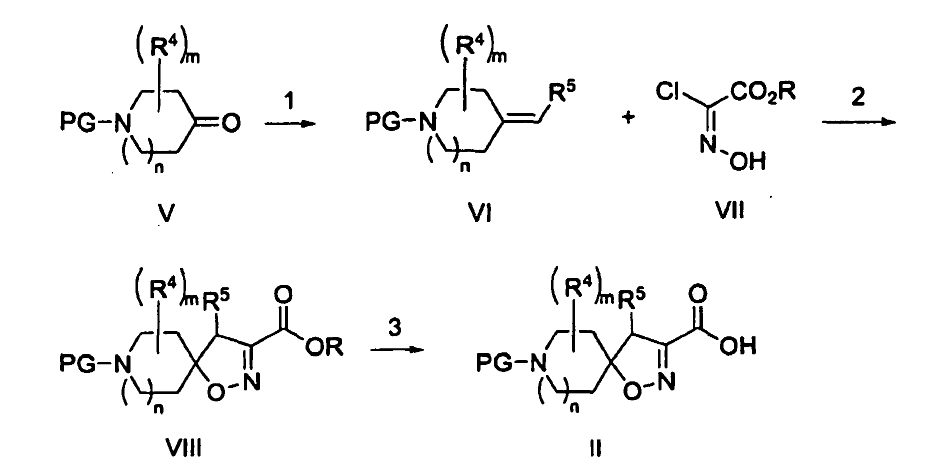

In Stufe 1 werden Verbindungen der allgemeinen Formel V, worin m, n, R⁴ und PG die vorstehend genannte Bedeutung haben, in einem Reaktionsmedium, vorzugsweise in einem Reaktionsmedium ausgewählt aus der Gruppe bestehend aus Tetrahydrofuran, Toluol, Diethylether und entsprechenden Mischungen, mit einem Reagenz zur Umwandlung von Carbonylgruppen In Doppelbindungen, bevorzugt mit einem Wittig-Reagenz der allgemeinen Formel R₃P(CH₂)R⁵X; worin R für einen Aryl-Rest steht, X für ein Halogenatom steht und R⁵ die vorstehend genannte Bedeutung hat; oder einem Wittig-Homer-Reagenz der allgemeinen Formel (RO)₂-P(=O)-(CH₂)-R⁵, worin R für einen Aryl-Rest steht und R⁵ die vorstehend genannte Bedeutung hat, besonders bevorzugt mit Methyltriphenylphosphoniumbromid, bei Temperaturen zwischen 0°C und 30 °C in Gegenwart einer Base, bevorzugt in Gegenwart eines Alkalimetallalkoholatsalzes, besonders bevorzugt in Gegenwart von Kalium-tert-butylat zu Verbindungen der allgemeinen Formel VI, worin m, n, R⁴, R⁵ und PG die vorstehend genannte Bedeutung haben, umgesetzt.

In Stufe 2 werden Verbindungen der allgemeinen Formel VI in einem Reaktionsmedium, bevorzugt in einem Reaktionsmedium ausgewählt aus der Gruppe bestehend aus Methanol, Tetrahydrofuran, Dichlormethan und entsprechenden Mischungen, in Gegenwart wenigstens einer Base, bevorzugt in Gegenwart von Natriumhydrogencarbonat, Lithiumhydroxid, Triethylamin oder N-Diisopropylethylamin, mit Verbindungen der allgemeinen Formel VII, worin R für einen linearen oder verzweigten C₁₋₆-Alkyl-Rest steht, besonders bevorzugt mit Verbindungen der allgemeinen Formel VII, worin R für einen Ethyl-Rest steht, d. h. mit Ethylchloroximidoacetat, bei Temperaturen zwischen 0 °C und 100 °C zu Verbindungen der allgemeinen Formel VIII, worin m, n, PG, R⁴, R⁵ und R die vorstehend genannte Bedeutung haben, umgesetzt.

In Stufe 3 werden Verbindungen der allgemeinen Formel VIII in einem Reaktionsmedium, bevorzugt in einem Reaktionsmedium ausgewählt aus der Gruppe bestehend aus Methanol, Ethanol, Wasser, Isopropanol und entsprechenden Mischungen, in Gegenwart wenigstens einer Base, bevorzugt in Gegenwart von Lithiumhydroxidmonohydrat, bei Temperaturen zwischen 0 °C und 50 °C zu Verbindungen der allgemeinen Formel **II** umgesetzt.

Die Verbindungen der vorstehend angegebenen Formeln R₃P(CH₂)R⁵X, (RO)₂-P(=O)-(CH₂)-R⁵, R¹-C(=O)-H, LG-R¹, LG-R⁷, LG-R⁹, HNR²R³, R⁶-N=C=O, R⁸-N=C=S, V und VII sind jeweils am Markt käuflich erhältlich und können auch nach üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten Spiro-Verbindungen der vorstehend genannten allgemeinen Formeln I und Ic, im Folgenden nur als Spiro-Verbindungen der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten Spiro-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen substituierten Spiro-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Spiro-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓP₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Spiro-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Spiro-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Spiro-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Spiro-Verbindung der vorstehend angegebenen allgemeinen Formel I, jeweils ggf. In Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, insbesondere zur Vanilloid-Rezeptor 1-(VR1/TRPV1)-Hemmung.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Haminkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes: Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit: Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation.

Bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Haminkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit: Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmelz, neuropathischem Schmerz und visceralem Schmerz, und/oder Harninkontinenz.

Bevorzugt ist die entsprechende Verwendung zur Herstellung eines Arzneimittels zur Prophylaxe und/oder Behandlung von Störungen oder Krankheiten, die zumindest teilweise durch Vanilloid-Rezeptoren 1 (VR1/TRPV1) vermittelt werden.

Besonders bevorzugt ist die entsprechende Verwendung zur Herstellung eines Arzneimittels zur Behandlung und/oder Prophylaxe von einer oder mehreren Erkrankungen ausgewählt aus der Gruppe bestehend aus akutem Schmerz; visceralem Schmerz; Gelenkschmerz; kognitiven Dysfunktionen, vorzugsweise kognitiven Mangelzuständen, besonders bevorzugt Gedächtnisstörungen; Lungenentzündung; Husten; einer überaktiven Blase (overactive bladder, OAB); Reizdarmsyndrom (irritable bowel syndrom); Augenreizungen; Hautreizungen; neurotischen Hauterkrankungen; Entzündungen des Darmes; Toleranzentwicklung gegenüber Medikamenten, vorzugsweise gegenüber natürlichen oder synthetischen Opioiden; und/oder zur Diurese oder zur Antinatriurese und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Das erfindungsgemäße Arzneimittel eignet sich zur Verabreichung an Erwachsene und Kinder einschließlich Kleinkindern und Säuglingen. Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern. Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten Spiro-Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen. Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten Spiro-Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße substituierte Spiro-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen erfindungsgemäßen substituierten Spiro-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzeiganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 mm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten inkubation werden 100 nM Capsaicin appliziert und ebenfalls der Einstrom von Ca²⁺ ermittelt.

Desensitisierende Agonisten und Antagonisten führten zu einer Unterdrückung des Ca²⁴-Einstroms. Es werden % Inhibierung im Vergleich zu der maximal erreichbaren Inhibierung mit 10 µM Capsazepin berechnet.

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen substituierten Verbindungen vor der Formalin-Injektion gewählt. Die intravenöse Applikation von 10 mg/kg Körpergewicht der Testsubstanzen erfolgt 5 Minuten vor der Formalin-injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten. Basierend auf der Quantifizierung des Schmerverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

## Patentansprüche

1. Substituierte Spiro-Verbindungen der allgemeinen Formel I, 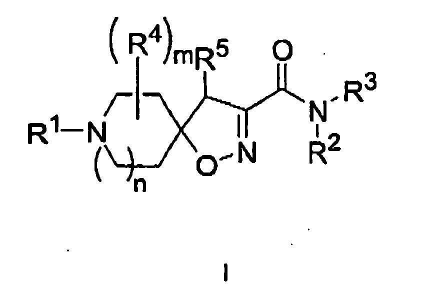 worin
m gleich 0, 1, 2, 3, oder 4 ist,
n gleich 0,
R¹ für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann,
für eine -C(=S)-NR⁸R⁹-Gruppe, oder
für -(CHR²⁶)-X_{q}-(CHR²⁷)ᵣ-Yₛ-(CHR²⁸)ᵣ₋Zᵤ-R²⁹ mit q = 0 oder 1, r = 0 oder 1, s = 0 oder 1, t = 0 oder 1, u = 0 oder 1, worin X, Y und Z, unabhängig voneinander, jeweils für O, S, NH, N(CH₃), N(C₂H₅) oder N[CH(CH₃)₂] stehen, oder
sofern m ungleich 0 und/oder n ungleich 1 und/oder R⁵ ungleich H ist, zusätzlich für eine -C(=O)-NR⁶R⁷-Gruppe stehen kann,
R² für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann,
oder für -(CHR³⁰)-Xᵥ(CHR³¹)_{w},Yₓ-(CHR³²)_{y}-Z_{z}-R³³ mit v = 0 oder 1, w = 0 oder 1,x = 0 oder 1,y = 0 oder 1,z = 0 oder 1, worin X, Y und Z, unabhängig voneinander, jeweils für O, S, NH, N(CH₃), N(C₂H₅) oder N[CH(CH₃)₂] stehen, steht;
R³ für einen Wasserstoff-Rest;
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrige cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann,
oder für -(CHR³⁰)-Xᵥ-(CHR³¹)_{w}-Yₓ-(CHR³²)_{y}-Z_{z}-R³³ mit v = 0 oder 1, w = 0 oder 1, x = 0 oder 1, y = 0 oder 1, z = 0 oder 1, worin X, Y und Z, unabhängig voneinander, jeweils für 0, S, NH, N(CH₃), N(C₂H₅) oder N[CH(CH₃)₂] stehen, steht;
oder
R² und R³ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, der mit einem gesättigten, ungesättigten oder aromatischen, ggf. substituierten mono- oder polyzyklischen Ringsystem kondensiert sein kann,
R⁴ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)- H-Gruppe, für eine Oxo-Gruppe (=O), für eine -NH-R¹⁰-Gruppe, für eine -NR¹¹R¹²-Gruppe, für eine -C(=O)-R³-Gruppe, für eine -C(=O)-O-R¹⁴- Gruppe, für eine -O-C(=O)-R¹⁵-Gruppe, für eine -NH-C(=O)-R¹⁶- Gruppe, für eine -NR¹⁷-C(=O)-R¹⁸-Gruppe, für eine -C(=O)-NH₂- Gruppe, für eine -C(=O)-NH-R¹⁹-Gruppe, für eine -C(=O)-NR²⁰R²¹- Gruppe, für eine -O-R²²-Gruppe, für eine -S-R²³-Gruppe, oder für einen linearen oder verzweigten, gesättigten oder ungesättigten, ggf. substituierten C₁₋₁₀ aliphatischen Rest steht;
R⁵ für einen Wasserstoff-Rest, für einen Halogen-Rest, für eine Nitro- Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo- Gruppe (=O), für eine -NH-R¹⁰-Gruppe, für eine -NR¹¹R¹²-Gruppe, für eine -C(=O)-R¹³-Gruppe, für eine -C(=O)-O-R¹⁴-Gruppe, für eine -O- C(=O)-R¹⁵-Gruppe, für eine -NH-C(=O)-R¹⁶-Gruppe, für eine -NR¹⁷- C(=O)-R¹⁸-Gruppe, für eine -C(=O)-NH₂-Gruppe, für eine -C(=O)-NH- R¹⁹-Gruppe, für eine -C(-O)-NR²⁰R²¹-Gruppe, für eine -O-R²²-Gruppe, für eine -S-R²³-Gruppe, für einen linearen oder verzweigten, gesättigten oder ungesättigten, ggf. substituierten C₁₋₁₀ aliphatischen Rest steht;
für einen ggf. substituierten 5- bis 14-gliedrigen Aryl- oder Heteroaryl- Rest
oder für -(CH₂)ₐₐ-R³⁴ mit aa = 1, 2, 3 oder 4 steht;
R⁶ und R⁸, unabhängig voneinander, jeweils
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen- Gruppen überbrückt sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH₂)ₑₑ-C(=O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-Zₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
R⁷ und R⁹, unabhängig voneinander, jeweils
für einen Wasserstoff-Rest;
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11-oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen- Gruppen überbrückt sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann;
für -(CHR³⁵)-(CH₂)_{bb}-(CH₂)_{cc}-C(=O)-R²⁴ mit bb = 0 oder 1 und cc = 0 oder 1;
für -(CHR³⁶)-(CH₂)_{dd}-(CH₂)ₑₑ-C(-O)-O-R²⁵ mit dd = 0 oder 1 und ee = 0 oder 1;
oder für -(CR³⁷R³⁸)-X_{ff}-(CHR³⁹)_{gg}-Yₕₕ-(CHR⁴⁰)ⱼⱼ-Zₖₖ-R⁴¹ mit ff = 0 oder 1, gg = 0 oder 1, hh = 0 oder 1, jj = 0 oder 1, kk = 0 oder 1, worin X, Y und Z, unabhängig voneinander, jeweils für O, S, NH, N(CH₃), N(C₂H₅) oder N[CH(CH₃)₂] stehen, stehen;
R¹⁰ bis R²⁵, unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl- Rest, der mit einem gesättigten oder ungesättigten, ggf. substituierten mono- oder polyzyklischen Ringsystem kondensiert sein kann,
oder für -(CH₂)ₘₘ-R⁴² mit mm gleich 1, 2 oder 3 stehen;
R²⁶, R²⁷, R²⁸, R³⁰, R³¹, R³², R³⁵, R³⁶, R³⁷, R³⁸, R³⁹ und R⁴⁰, unabhängig voneinander, jeweils
für einen Wasserstoff-Rest;
für einen linearen oder verzweigten, gesättigten oder ungesättigten, ggf. substituierten C₁₋₁₀ aliphatischen Rest,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
R²⁹, R³³ und R⁴¹, unabhängig voneinander, jeweils
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit 1, 2, 3, 4 oder 5 linearen oder verzweigten, ggf. substituierten C₁₋₅-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
und
R³⁴ und R⁴², unabhängig voneinander, jeweils
für einen ggf. substituierten 5- bis 14-gliedrigen Aryl- oder Heteroaryl- Rest stehen;
wobei
die vorstehend genannten C₁₋₁₀ aliphatischen Reste und C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, - NO₂, -OH, -SH und -NH₂ substituiert sein können;
die vorstehend genannten cycloallphatischen Reste und heterocycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂,-O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, - S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, - O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -C₁₋₅-Alkyl, -O-C₁₋₅-Alkyl, -O-CF₃, -S-CF₃, Phenyl und -O-Benzyl, substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten heterocycloaliphatischen Reste jeweils ggf. 1, 2 oder 3 zusätzliche(s) Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(-O)-C₁₋₅-Alkyl, -C(=O)-NH₂, -C(=O)-NH-C₁₋₅-Alkyl, C(=O)-N-(C₁₋₅=Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, - NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(-O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, - S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
R¹ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂N₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexehyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazollnyl steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazollnyl, Chinolinyl, Isochinolinyl, Benzimidazolinyl, Benzoxazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H. -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H_{5,} -O-CF₃, -S-CF₃, Phenyl und -O-Benzyl substituiert sein kann;
für eine -C(=S)-NR⁸R⁹-Gruppe steht; oder
für -(CHR²⁶)-R²⁹; (CHR²⁶)-(CHR²⁷)-R²⁹; -(CHR²⁶)-(CHR²⁷)-O-R²⁹; -(CHR²⁶)-(CHR²⁷)-(CHR²⁸)-R²⁹; -(CHR²⁶)-(CHR²⁷)-S-(CHR²⁸)-R²⁹; -(CHR²⁶)-(CHR²⁷)-(CHR²⁸)-N(CH₃)-R²⁹ steht, oder
sofern m ungleich 0 und/oder n ungleich 1 und/oder R⁵ ungleich H ist, zusätzlich für eine -C(=O)-NR⁶R⁷-Gruppe stehen kann,

3. Verbindungen gemäß Anspruche 1 bis 2, **dadurch gekennzeichnet, dass**
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂,-C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für -(CHR³⁰)-R³³; -(CHR³⁰)-(CHR³¹)-R³³; -(CHR³⁰)-(CHR³¹)-O-R³³; - (CHR³⁰)-(CHR³¹)-(CHR³²)-R³³; -(CHR³⁰)-(CHR³¹)-S-(CHR³²)-R³³; -(CHR³⁰)-(CHR³¹)-(CHR³²)-N(CH₃)-R³³ steht.

4. Verbindungen gemäß einem oder mehreren der Ansprüche 1 bis 3, **dadurch gekennzeichnet, dass**
R³ für einen Wasserstoff-Rest steht.

5. Verbindungen gemäß einem oder mehreren der Ansprüche 1 bis 4, **dadurch gekennzeichnet, dass**
R² und R³ zusammen mit dem sie verbindenden Stickstoffatom als Ringglied einen Rest bilden ausgewählt aus der Gruppe bestehend aus Pyrrolidinyl, Piperidinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl, (1,3)-Thiazolidinyl, Piperazinyl, Morpholinyl, Azepanyl, Diazepanyl und Thiomorpholinyl bilden, wobei der Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -0-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)_{3,} -N(CH₃)_{2,} -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituierte sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅. -O-CF₃, -S-CF₃, Phenyl und -O-Benzyl substituiert sein kann.

6. Verbindungen gemäß einem oder mehreren der Ansprüche 1 bis 5, **dadurch gekennzeichnet, dass**
R⁴ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann.

7. Verbindungen gemäß einem oder mehreren der Ansprüche 1 bis 6, **dadurch gekennzeichnet, dass**
R⁵ für einen Wasserstoff-Rest steht,
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-H, -C(=O)-CH₃,-C(=O)-C₂H₅, -C(=O)-C(CH₃)₃, -C(=O)-NH₂; -C(=O)-NH-CH₃, -C(=O)-NH-C₂H₅, -C(=O)-N-(CH₃)₂ und -C(=O)-N-(C₂H₅)₂ substituiert sein kann;
oder für -(CH₂)-R³⁴, -(CH²)-(CH²)-R³⁴ oder -(CH₂)-(CH₂)-(CH₂)-R³⁴ steht.

8. Verbindungen gemäß einem oder mehreren der Ansprüche 1 bis 7, **dadurch gekennzeichnet, dass**
R⁶ und R⁸, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, - NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CN₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, - S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)-CH₃, -S(=O)₂C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CHR³⁵)-C(=O)-R²⁴ oder -(CHR³⁵)-(CH₂)-C(=O)-R²⁴ stehen;
für -(CHR³⁶)-C(=O)-O-R²⁵ oder -(CHR³⁶)-(CH₂)-C(=O)-O-R²⁵ stehen;
oder für -(CR³⁷R³⁸)-R⁴¹, -(CR³⁷R³⁸)-(CHR³⁹)-R⁴¹, -(CR³⁷R³⁸)-(CHR³⁹)-O-R⁴¹, - (CR³⁷R³⁸)-(CHR³⁹)-(CHR⁴⁰)-R⁴¹, -(CR³⁷R³⁸)-(CHR³⁹)-(CHR⁴⁰)-O-R⁴¹, - (CR³⁷R³⁸)-(CHR³⁹)-(CH^{R40})-(CH₃)-R⁴¹ oder -(CR³⁷R³⁸)-(CHR³⁹)-(CHR⁴⁰)-N(C₂H₅)-R⁴¹ stehen.

9. Verbindungen gemäß einem oder mehreren der Ansprüche 1 bis 8, **dadurch gekennzeichnet, dass**
R⁷ und R⁹ jeweils für einen Wasserstoff-Rest stehen.

10. Verbindungen gemäß einem oder mehreren der Ansprüche 1 bis 9, **dadurch gekennzeichnet, dass**
R¹⁰ bis R²⁵, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen,
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest jeweils mit 1 oder 2 Substituenten unabhängig voneinander ausgewählt aus der Gruppe bestehend aus -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, Pyridinyl, Phenyl und Benzyl substituiert sein kann, wobei jeweils der zyklische Teil der Reste Pyridinyl, -S(=O)₂-Phenyl, Phenyl und Benzyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, -O-CH₃ und -O-C₂H₅ substituiert sein kann;
für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann,
oder für -(CH₂)-R⁴² oder -(CH₂)-(CH₂)-R⁴² stehen.

11. Verbindungen gemäß einem oder mehreren der Ansprüche 1 bis 10, **dadurch gekennzeichnet, dass**
R²⁶, R²⁷, R²⁸, R³⁰, R³¹, R³², R³⁵, R³⁶, R³⁷, R³⁸, R³⁹ und R⁴⁰, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃; -C(=O)-O-C₂H₅, -C(=O)-O-C(CH₃)₃, -O-C(=O)-CH₃, -O-C(=O)-C₂H₅, - O-C(=O)-C(CH₃)₃, -N(CH₃)₂, -N(C₂H₅)₂, -NH-CH₃, -NH-C₂H₅, -C(=O)-CH₃, - C(=O)-C₂H₅, -C(=O)-C(CH₃)₃, Cyclohexyl, Cyclopentyl, -O-Phenyl, -O-Benzyl und Phenyl substituiert sein kann.

12. Verbindungen gemäß einem oder mehreren der Ansprüche 1 bis 11, **dadurch gekennzeichnet, dass**
R²⁹, R³³ und R⁴¹, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, isopropyl, n-Butyl, sec-Butyl, Isobutyl, tert-Buty, -C(=O)-OH, -C(=O)-O-CH₃, -C(=O)-O-C₂H₅, -C(=O)-O-C(CH₃)₃, -0-C(=O)-CH₃, -O-C(=O)C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, C(=O)-H, -C(=O)-CH₃, -C(=O)-C₂H₅, -C(=O)-C(CH₃)₃, -C(=O)-NH₂, - C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-Phenyl, - -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃. Phenyl und -O-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -(7-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H5)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl -O-Phenyl, -0-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

13. Verbindungen gemäß einem oder mehreren der Ansprüche 1 bis 12, **dadurch gekennzeichnet, dass**
R³⁴ und R⁴², unabhängig voneinander, jeweils für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, - C(=O)-C(CH₃)₃ substituiert sein kann.

14. Verbindungen gemäß einem oder mehreren der Ansprüche 1 bis 13, **dadurch gekennzeichnet, dass**
m gleich 0 ist.

15. Verbindungen gemäß einem oder mehreren der Ansprüche 1 bis 14, **dadurch gekennzeichnet, dass** die Verbindungen im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

16. Verfahren zur Herstellung von substituierten Spiro-Verbindungen der allgemeinen Formel I gemäß einem oder mehreren der Ansprüche 1 bis 15, **dadurch gekennzeichnet, dass** wenigstens eine Verbindung der allgemeinen Formel II, 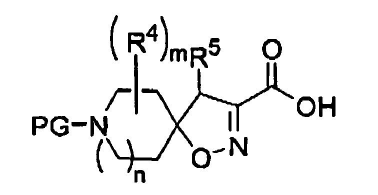 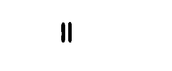 worin R⁴, R⁵, m und n die Bedeutung gemäß einem oder mehreren der Ansprüche 1 bis 15 haben und PG für eine Schutzgruppe, bevorzugt für eine Benzyloxycarbonyl- oder tert-Butyloxycarbonyl-Gruppe, steht, in einem Reaktionsmedium In Gegenwart wenigstens eines Kupplungsreagenzes ggf. In Gegenwart wenigstens einer Base mit einer Verbindung der allgemeinen Formel HNR²R³, worin R² und R³ die Bedeutung gemäß einem oder mehreren der Ansprüche 1 bis 15 haben, zu wenigstens einer Verbindung der allgemeinen Formel III, 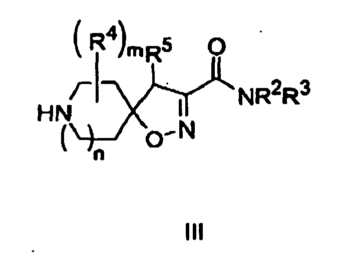 worin R², R³, R⁴, R⁵, m, n und PG die vorstehend genannte Bedeutung haben, umgesetzt wird und diese ggf. gereinigt und/oder isoliert wird
und
wenigstens eine Verbindung der allgemeinen Formel III in einem Reaktionsmedium In Gegenwart wenigstens einer Säure, vorzugsweise in Gegenwart wenigstens einer anorganischen oder organischen Säure ausgewählt aus der Gruppe bestehend aus Salzsäure, Schwefelsäure, Essigsäure und Trifluoressigsäure, oder in Gegenwart von Wasserstoff und eines Katalysators, vorzugsweise eines Katalysators basierend auf Palladium oder Platin, zu wenigstens einer Verbindung der allgemeinen Formel IV, 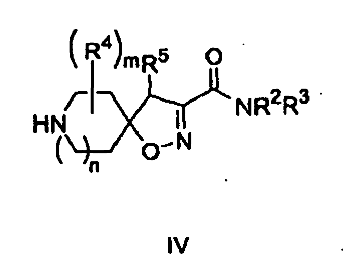 worin R², R³, R⁴, R⁵, m und n die vorstehend genannte Bedeutung haben, umgesetzt wird und diese ggf. gereinigt und/oder isoliert wird
und
wenigstens eine Verbindung der allgemeinen Formel IV in einem Reaktionsmedium mit wenigstens einem Isocyanat der allgemeinen Formel R⁶-N=C=O, worin R⁶ die Bedeutung gemäß einem oder mehreren der Ansprüche 1 bis 15 hat, ggf. in Gegenwart wenigstens einer Base, bevorzugt in Gegenwart wenigstens einer Base ausgewählt aus der Gruppe bestehend aus Triethylamin, 4.4-Dimethylaminopyridin und Dilsopropylethylamin, zu wenigstens einer Verbindung der allgemeinen Formel 1 umgesetzt wird, worin R⁷, R³, R⁴, R⁵, m und n die vorstehend genannte Bedeutung haben und R¹ für -C(=O)-NF⁶F⁷ steht, wobei R⁶ die vorstehend genannte Bedeutung hat und R⁷ für einen Wasserstoff-Rest steht, und diese ggf. gereinigt und/oder isoliert wird
oder
wenigstens eine Verbindung der allgemeinen Formel IV In einem Reaktionsmedium mit wenigstens einem Isothiocyanat der allgemeinen Formel S=C=N-R⁸ worin R⁸ die Bedeutung gemäß einem oder mehreren der Ansprüche 1 bis 15 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R², R³, R⁴, R⁵, m und n die vorstehend genannte Bedeutung haben und R¹ für -C(=S)-N-R⁸R⁹ steht, wobei R⁸ die vorstehend genannte Bedeutung hat und R⁹ für einen Wasserstoff-Rest steht, und diese ggf. gereinigt und/oder isoliert wird
und ggf. wenigstens eine Verbindung der allgemeinen Formel I, worin R², R³, R⁴, R⁵, m und n die vorstehend genannte Bedeutung haben und R¹ für - C(=O)-NR⁶R⁷ oder -C(=S)N-R⁸R⁹ steht, worin R⁷ und R⁹ jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydrtdsalzes oder eines Metalialkoholatsalzes ausgewählt aus der Gruppe bestehend aus Natriumhydrid, Kaliumhydrid, Kalium-tert-butanolat. Natium-tert-butanolat, Kaliummethanolat, Natriummethanolat, Natriumethanolat und Kaliumethanolat, mit wenigstens einer Verbindung der allgemeinen Formel LG-R⁷ oder der allgemeinen Formel LG-R⁹, worin LG für eine Abgangsgruppe, bevorzugt für ein Halogen-Atom, besonders bevorzugt für ein Chloratom steht, und R⁷ und R⁹ die , vorstehend genannte Bedeutung mit Ausnahme von Wasserstoff haben, zu wenigstens einer Verbindung der allgemeinen Formel I umgesetzt wird, worin R¹ bis R⁵, m und n die vorstehend genannte Bedeutung haben, und diese ggf. gereinigt und/oder isoliert wird,
oder
wenigstens eine Verbindung der allgemeinen Formel IV in einem Reaktionsmedium, in Gegenwart wenigstens einer Base, bevorzugt In Gegenwart wenigstens eines Metallhydridsalzes, besonders bevorzugt in Gegenwart von Natrium- und/oder Kaliumhydrid, mit wenigstens einer Verbindung der allgemeinen Formel LG-R¹, worin R¹ die Bedeutung gemäß einem oder mehreren der Ansprüche 1 bis 15 hat, mit Ausnahme von - C(=O)-NR⁶R⁷ und -C(=S)-N⁸R⁹, und LG für eine Abgangsgruppe, bevorzugt für ein Halogen-Atom, besonders bevorzugt für ein Chloratom steht, zu wenigstens einer Verbindung der allgemeinen Formel **I** umgesetzt wird, worin R¹bis R⁵, m und n die vorstehend genannte Bedeutung haben, und diese ggf. gereinigt und/oder isoliert wird
oder
wenigstens eine Verbindung der allgemeinen Formel IV in einem Reaktionsmedium, in Gegenwart wenigstens eines Reduktionsmittels, mit wenigstens einer Verbindung der allgemeinen Formel R¹-C(=O)-H, worin R¹ die Bedeutung gemäß einem oder mehreren der Ansprüche 1 bis 15 hat, mit Ausnahme von -C(=O)-NR⁶R⁷ und -C(=O)-NR⁸R⁹, zu wenigstens einer Verbindung der allgemeinen Formel **I** umgesetzt wird, worin R¹ bis R⁶, m und n die vorstehend genannte Bedeutung haben, und diese ggf. gereinigt und/oder isoliert wird.

17. Arzneimittel enthaltend wenigstens eine Verbindung gemäß einem oder mehreren der Ansprüche 1 bis 15 und ggf. einen oder mehrere physiologisch verträgliche Hilfsstoffe.

18. Arzneimittel gemäß Anspruch 17 zur Prophylaxe und/oder Behandlung von Schmerz, vorzugsweise von Schmerz ausgewählt aus der Gruppe bestehend aus akutem Schmerz, chronischem Schmerz, neuropathischem Schmerz, und visceralem Schmerz

19. Arzneimittel gemäß Anspruch 17 zur zur Behandlung und/oder Prophylaxe von einer oder mehreren Erkrankungen, ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; loleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

20. Verwendung wenigstens einer Verbindung gemäß einem oder mehreren der Ansprüche 1 bis 15 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

21. Verwendung wenigstens einer Verbindung gemäß einem oder mehreren der Ansprüche 1 bis 15 zur Herstellung eines Arzneimittels zur Prophylaxe und/oder Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit, Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse, zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

## Claims

1. Substituted spiro-compounds of the general formula I, 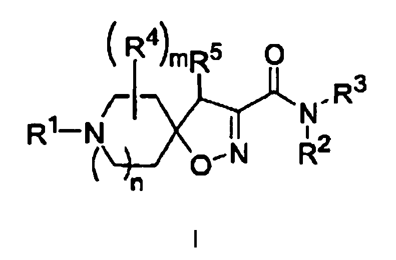 wherein
m equals 0, 1, 2, 3, or 4,
n equals 0,
R¹ is a linear or branched, saturated or unsaturated, possibly substituted C₁₋₁₀ aliphatic residue;
is an unsaturated or saturated, possibly substituted 3, 4, 5, 6, 7, 8, or 9 member cycloaliphatic residue that may be condensed with a saturated, unsaturated, or aromatic, possibly substituted mono or polycyclic ring system;
is a possibly substituted 5 to 14 member aryl or heteroaryl residue that may be condensed with a saturated or unsaturated, possibly substituted mono or polycyclic ring system,
is a -C(=S)-NR⁸R⁹ group, or
is -(CHR²⁶)-X_{q}-(CHR²⁷)ᵣ-Yₛ-(CHR²⁸)ₜ- Zᵤ-R²⁹ with q = 0 or 1, r = 0 or 1, s = 0 or 1, t = 0 or 1, u = 0 or 1, wherein X, Y, and Z each independently of each other are O, S, NH, N(CH₃), N(C₂H₅), or N[CH(CH₃)₂], or
if m is not equal to 0 and/or if n is not equal to 1 and/or if R⁵ is not equal to H, the same may also be a -C(=O)-NR⁶R⁷ group,
R² is a linear or branched, saturated or unsaturated, possibly substituted C₁₋₁₀ aliphatic residue;
is an unsaturated or saturated, possibly substituted 3, 4, 5, 6, 7, 8, or 9 member cycloaliphatic residue that may be condensed with a saturated, unsaturated, or aromatic, possibly substituted mono or polycyclic ring system;
is a possibly substituted 5 to 14 member aryl or heteroaryl residue that may be condensed with a saturated or unsaturated, possibly substituted mono or polycyclic ring system,
or is -(CHR³⁰)-Xᵥ-(CHR³¹)_{w}-Yₓ-(CHR³²)_{y}-Z_{z}-R³³ with v = 0 or 1, w = 0 or 1, x = 0 or 1, y = 0 or 1, z = 0 or 1, wherein X, Y, and Z are each independently of each other O, S, NH, N(CH₃), N(C₂H₅) or N[CH(CH₃)₂];
R³ is a hydrogen residue;
is a linear or branched, saturated or unsaturated, possibly substituted C₁₋₁₀ aliphatic residue;
is an unsaturated or saturated, possibly substituted 3, 4, 5, 6, 7, 8, or 9 member cycloaliphatic residue that may be condensed with a saturated, unsaturated, or aromatic, possibly substituted mono or polycyclic ring system;
is a possibly substituted 5 to 14 member aryl or heteroaryl residue that may be condensed with a saturated or unsaturated, possibly substituted mono or polycyclic ring system,
or is -(CHR³⁰)-Xᵥ-(CHR³¹)_{w}-Yₓ-(CHR³²)_{y}-Z_{z}-R³³ with v = 0 or 1, w = 0 or 1, x = 0 or 1, y = 0 or 1, z = 0 or 1, wherein X, Y, and Z are each independently of each other O, S, NH, N(CH₃), N(C₂H₅) or N[CH(CH₃)₂];
or
and R³, together with the nitrogen atom connecting the same as a ring member, form a saturated or unsaturated, possibly substituted 4, 5, 6, 7, 8, or 9 member heterocycloaliphatic residue that may be condensed with a saturated, unsaturated, or aromatic, possibly substituted mono or polycyclic ring system,
R⁴ is a halogen residue, a nitro-group, a cyano-group, an amino-group, a hydroxy group, a thiol group, a carboxy group, a formyl group, an -NH-C(=O)-H group, an oxo-group (=O), an -NH-R¹⁰ group, an -NR¹¹R¹² group, a -C(=O)-R¹³ group, a -C(=O)-O-R¹⁴ group, an -O-C(=O)-R¹⁵ group, an -NH-C(=O)-R¹⁶ group, an -NR¹⁷- C(=O)-R¹⁸ group, a -C(=O)-NH₂ group, a -C(=O)-NH-R¹⁹ group, a -C(=O)-NR²⁰R²¹ group, an -O-R²² group, a -S-R²³ group, or a linear or branched, saturated or unsaturated, possibly substituted C₁₋₁₀ aliphatic residue;
R⁵ is a hydrogen residue, a halogen residue, a nitro-group, a cyano-group, an amino-group, a hydroxy group, a thiol group, a carboxy group, a formyl group, an -NH-C(=O)-H group, an oxo-group (=O), an -NH-R¹⁰ group, an -NR¹¹R¹² group, a -C(=O)-R¹³ group, a -C(=O)-O-R¹⁴ group, an -O-C(=O)-R¹⁵ group, an -NH-C(=O)- R¹⁶ group, an -NR¹⁷-C(=O)-R¹⁸ group, a -C(=O)-NH₂ group, a -C(=O)-NH-R¹⁹ group, a -C(=O)-NR²⁰R²¹ group, an -O-R²² group, a -S-R²³ group, or a linear or branched, saturated or unsaturated, possibly substituted C₁₋₁₀ aliphatic residue;
is a possibly substituted 5 to 14 member aryl or heteroaryl residue,
or is -(CH₂)ₐₐ-R³⁴ wherein aa = 1, 2, 3, or 4;
R⁶ and R⁸ are each independently of each other
a linear or branched, saturated or unsaturated, possibly substituted C₁₋₂₀ aliphatic residue;
an unsaturated or saturated, possibly substituted 3, 4, 5, 6, 7, 8, 9, 10, 11, or 12 member cycloaliphatic residue that may be condensed with a saturated, unsaturated, or aromatic, possibly substituted mono or polycyclic ring system and/or bridged by means of one or two linear or branched, possibly substituted C₁₋₅ alkylene groups;
a possibly substituted 5 to 14 member aryl or heteroaryl residue that may be condensed with a saturated or unsaturated, possibly substituted mono or polycyclic ring system;
-(CHR³⁵)-(CH₂)_{bb}-(CH₂)_{cc}-C(=O)-R²⁴ wherein bb = 0 or 1, and cc = 0 or 1;
-(CHR³⁶⁾-(CH2)_{dd-}(CH₂)ₑₑ-C(=O)-O-R²⁵ wherein dd = 0 or 1, and ee = 0 or 1;
or -(CR³⁷R³⁸)-X_{ff}-(CHR³⁹)_{gg}-Yₕₕ-(CHR⁴⁰)-Zₖₖ-R⁴¹ wherein ff = 0 or 1, gg = 0 or 1, hh = 0 or 1, jj = 0 or 1, kk = 0 or 1, wherein X, Y, and Z are each independently of each other O, S, NH, N(CH₃), N(C₂H₅), or N[CH(CH₃)₂];
R⁷and R⁹ are each independently of each other
a hydrogen residue;
a linear or branched, saturated or unsaturated, possibly substituted C₁₋₂₀ aliphatic residue;
an unsaturated or saturated, possibly substituted 3, 4, 5, 6, 7, 8, 9, 10, 11, or 12 member cycloaliphatic residue that may be condensed with a saturated, unsaturated, or aromatic, possibly substituted mono or polycyclic ring system and/or bridged by means of one or two linear or branched, possibly substituted C₁₋₅ alkylene groups;
a possibly substituted 5 to 14 member aryl or heteroaryl residue that may be condensed with a saturated or unsaturated, possibly substituted mono or polycyclic ring system;
-(CHR³⁵)-(CH₂)_{bb}-(CH₂)_{cc}-C(=O)-R²⁴ wherein bb = 0 or 1, and cc = 0 or 1;
-(CHR³⁶)-(CH₂)_{dd}-(CH₂)ₑₑ-C(=O)-O-R²⁵ wherein dd = 0 or 1, and ee = 0 or 1;
or -(CR³⁷R³⁸)-X_{ff}-(CHR³⁹)_{gg}-Yₕₕ-(CHR⁴⁰)jj-Zₖₖ-R⁴¹ wherein ff = 0 or 1, gg = 0 or 1, hh = 0 or 1, jj = 0 or 1, kk = 0 or 1, wherein X, Y, and Z are each independently of each other O, S, NH, N(CH₃), N(C₂H₅), or N[CH(CH₃)₂];
R¹⁰ to R²⁵ are each independently of each other
a linear or branched, saturated or unsaturated, possibly substituted C₁₋₁₀ aliphatic residue;
an unsaturated or saturated, possibly substituted 3, 4, 5, 6, 7, 8, or 9 member cycloaliphatic residue that may be condensed with a saturated, unsaturated, or aromatic, possibly substituted mono or polycyclic ring system;
a possibly substituted 5 to 14 member aryl or heteroaryl residue that may be condensed with a saturated or unsaturated, possibly substituted mono or polycyclic ring system;
or -(CH₂)ₘₘ-R⁴² wherein mm equals 1, 2, or 3;
p²⁶ R²⁷, R²⁸, R³⁰, R³¹, R³², R³⁵, R³⁶, R³⁷, R³⁸, R³⁹ and R⁴⁰ are each independently of each other
a hydrogen residue;
a linear or branched, saturated or unsaturated, possibly substituted C₁₋₁₀ aliphatic residue,
or a possibly substituted 5 to 14 member aryl or heteroaryl residue that may be condensed with a saturated or unsaturated, possibly substituted mono or polycyclic ring system;
R²⁹, R³³ and R⁴¹ are each independently of each other
a linear or branched, saturated or unsaturated, possibly substituted C₁₋₁₀ aliphatic residue;
an unsaturated or saturated, possibly substituted 3, 4, 5, 6, 7, 8, or 9 member cycloaliphatic residue that may be bridged by means of 1, 2, 3, 4, or 5 linear or branched, possibly substituted C₁₋₅ alkylene groups and/or condensed with a saturated, unsaturated, or aromatic, possibly substituted mono or polycyclic ring system; or
a possibly substituted 5 to 14 member aryl or heteroaryl residue that may be condensed with a saturated or unsaturated, possibly substituted mono or polycyclic ring system;
and
R³⁴ and R⁴² are each independently of each other
a possibly substituted 5 to 14 member aryl or heteroaryl residue;
wherein
the C₁₋₁₀ aliphatic residues and C₁₋₂₀ aliphatic residues stated above may each possibly be substituted by 1, 2, 3, 4, 5, 6, 7, 8, or 9 substituents, independently selected from the group consisting of F, Cl, Br, I, -CN, -NO₂, -OH, -SH, and -NH₂,
the cycloaliphatic residues and heterocycloaliphatic residues stated above may possibly be substituted by 1, 2, 3, 4, or 5 substituents, independently selected from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, - C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl, and benzyl, wherein each cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may possibly substituted by 1, 2, 3, 4, or 5 substituents, independently selected from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the cycloaliphatic residues stated above may possibly have 1, 2, 3, 4, or 5 heteroatom(s), independently selected from the group consisting of oxygen, nitrogen, and sulfur as ring member(s);
the heterocycloaliphatic residues stated above may possibly have 1, 2, or 3 additional heteroatom(s), independently selected from the group consisting of oxygen, nitrogen, and sulfur as ring member(s);
the rings of the above stated mono or polycyclic ring systems may possibly be substituted by 1, 2, 3, 4, or 5 substituents, independently selected from the group consisting of oxo (=O), thioxo (=S), F, Cl, Br, I, -CN, -CF₃, -SF₅, -OH, -O-C₁₋₅-alkyl, - NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, - C(=O)-H, -C(=O)-C₁₋₅-alkyl, -C(=O)-NH₂, -C(=O)-NH-C₁₋₅-alkyl, C(=OFN-(C₁₋₅-alkyl)₂, - S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl, and benzyl, wherein each cyclic part of the residues of pyridinyl, cyclopentyl, cyclohexyl, pyridazinyl, -S(=O)₂-phenyl, -O-phenyl, -O-benzyl, phenyl, - (CH₂)-benzo[b]furanyl and benzyl may possibly be substituted by 1, 2, 3, 4, or 5 substituents, independently selected from the groups consisting of F, Cl, Br, -OH, -CF₃, - SF₅, -CN, -NO₂, -C₁₋₅-alkyl, -O-C₁₋₅-alkyl, -O-CF₃, -S-CF₃, phenyl and -O-benzyl,
and the rings of the above stated mono or polycyclic ring systems each have 5, 6, or 7 members, and may possibly each have 1, 2, 3, 4, or 5 heteroatom(s) as ring member(s) that are independently selected from the group of oxygen, nitrogen, and sulfur;
and the above stated aryl or heteroaryl residues may possibly each be substituted by 1, 2, 3, 4, or 5 substituents, independently selected from the group consisting of F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁. ₅-alkyl, C(=O)-N-(C₁₋₅-alkyl)₂, -S(=O)₂-C₁₋₅- alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl, and benzyl, wherein each cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may possibly be substituted by 1, 2, 3, 4, or 5 substituents, independently selected from the groups consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -0-benzyl,
the heteroaryl residues stated above may possibly have 1, 2, 3, 4, or 5 heteroatom(s), independently selected from the group consisting of oxygen, nitrogen, and sulfur as ring element(s);
and
the C₁₋₅-alkylene groups stated above may possibly be substituted by 1, 2, 3, 4, or 5 substituents, independently selected from the group consisting of F, Cl, Br, -OH, -SH, - NH₂, -CN and NO₂;
each may possibly be in the form of a pure stereoisomer thereof, particularly an enantiomer or a diastereomer, the racemate thereof, or in the form of a mixture of stereoisomers, particularly of the enantiomers, and/or diastereomers, in any desired mixture ratio, or each in the form of respective salts, or each in the form of respective solvates.

2. The compounds according to claim 1, **characterized in that**
R¹ is a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl, and 3-butinyl, wherein the residue may possibly be substituted by 1, 2, 3, 4, or 5 substituents, independently selected from the group consisting of F, Cl, Br, I, -CN, - NO₂, -OH, -SH, and -NH₂;
is a residue, selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl, and (1,2,3,4)-tetrahydroquinazolinyl, wherein the residue may possibly be substituted by 1, 2, 3, 4, or 5 substituents, independently selected from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, - C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may each be substituted by 1, 2, 3, 4, or 5 substituents, independently selected from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -0-benzyl;
is a residue, selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl, and benzothiazolyl, wherein the residue may possibly be substituted by 1, 2, 3, 4, or 5 substituents, independently selected from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=OFC₂H₅, -O-C(=O)-C(CH₃)₃, -N(CH₃)₂, -N(C₂H₅)₂, -NH-CH₃, -NH-C₂H₅, -NH-C(=O)-O-CH_{3.} -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, - C(=O)-NH₂, -C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phenyl, -NH-S(=O)₂-CH₃, -NH-S(=O)₂-C₂H₅, - S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may each be substituted by 1, 2, 3, 4, or 5 substituents, independently selected from the group consisting of F, Cl, Br, -OH, - CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl;
is a -C(=S)-NR⁸R⁹-group; or
is -(CHR²⁶)-R²⁹; -(CHR²⁶)-(CHR²⁷)-R²⁹; -(CHR²⁶)-(CHR²⁷)-O-R²⁹; -(CHR²⁶)-(CHR²⁷)-(CHR²⁸)-R²⁹; -(CHR²⁶)(CHR²⁷)-S-(CHR²⁸)-R²⁹;-(CHR²⁶)-(CHR²⁷)-(CHR²⁸)-N(CH₃)R²⁹, or
if m is not equally 0 and/or n is not equally 1 and/or R⁵ is not equally H, may additionally be a -C(=O)-NR⁶R⁷ group.

3. The compounds according to claims 1 to 2, **characterized in that**
R² is a residue, selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)- (CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl, and 3-butinyl, wherein the residue may possibly be substituted by 1, 2, 3, 4, or 5 substituents, independently selected from the group consisting of F, Cl, Br, I, - - CN, -NO₂, -OH, -SH, and -NH₂;
is a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl, and (1,2,3,4)-tetrahydroquinazolinyl, wherein the residue may possibly each be substituted by 1, 2, 3, 4, or 5 substituents, independently selected from the group consisting of oxo (=O), thioxo (=S), F, Cl, Br, I, -CN, -CF₃, -SF₅, -OH, -O-CH₃, -O-C₂H₅, -NH₂, -NO₂, -0-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may each be substituted by 1, 2, 3, 4, or 5 substituents, independently selected from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl;
is a residue selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl,
imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl, and benzothiazolyl, wherein the residue may possibly be substituted by 1, 2, 3, 4, or 5 substituents, independently selected from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=OFC₂H₅, -C(=O)-C(CH₃)_{3,} - C(=O)-NH₂, -C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phenyl, -NH-S(=O)₂-CH₃, -NH-S(=O)₂-C₅H₅, - S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -0-benzyl, phenyl, -(CH₂)-benzo[b]furanyl and benzyl may each be substituted by 1, 2, 3, 4, or 5 substituents, independently selected from the group consisting of F, Cl, Br, -OH, - CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl;
or is -(CHR³⁰)R³³; -(CHR³⁰)-(CHR³¹)-R³³; -(CHR³⁰)-(CHR³¹)-O-R³³; -(CHR³⁰)-(CHR³¹)-(CHR³²)-R³³; -(CHR³⁰)-(CHR³¹)-S-(CHR³²)-R³³;-(CHR³⁰)-(CHR³¹)-(CHR³²)-N(CH₃)-R³³.

4. The compounds according to one or several of the claims 1 to 3, **characterized in that**
R³ is a hydrogen residue.

5. The compounds according to one or several of the claims 1 to 4, **characterized in that**
R² and R³, together with the nitrogen atom connecting the same as a ring member, form a residue, selected from the group consisting of pyrrolidinyl, piperidinyl, (1,3,4,5)-tetrahydropyrido[4,3-b]indolyl, (3,4)-dihydro-1H-isoquinolinyl, (1,3,4,9)-tetrahydro-[b]-carbolinyl, Imidazolidinyl, (1,3)-thiazolidinyl, piperazinyl, morpholinyl, azepanyl, diazepanyl, and thiomorpholinyl, wherein the residue may be substituted by 1, 2, 3, 4, or 5 substituents, independently selected from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, - SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O}-O-C₂H₅, -C(=O)-O-C(CH₃)₃, -O-C(=O)-CH₃, - O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, - C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may be substituted by 1, 2, 3, 4, or 5 substituent, independently selected from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl.

6. The compounds according to one or several of the claims 1 to 5, **characterized in that**
R⁴ is a residue selected from the group consisting of methyl, ethyl, n-propyl, isopropyl, n-butyl, sec-butyl, isobutyl, tert-butyl, n-pentyl, sec-pentyl, n-hexyl, and n-heptyl, wherein the residue may possibly be substituted by 1, 2, 3, 4, or 5 substituents, independently selected from the group consisting of F, Cl, Br, I, -CN, -NO₂, -OH, -SH, and -NH₂.

7. The compounds according to one or several of the claims 1 to 6, **characterized in that**
R⁵ is a hydrogen residue,
is a residue selected from the group consisting of methyl, ethyl, n-propyl, isopropyl, n-butyl, sec-butyl, isobutyl, tert-butyl, n-pentyl, sec-pentyl, n-hexyl, and n-heptyl, wherein the residue may possibly be substituted by 1, 2, 3, 4, or 5 substituents, independently selected from the group consisting of F, Cl, Br, I, -CN, -NO₂, -OH, -SH, and -NH₂;
is a residue selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl, and benzothiazolyl, wherein the residue may possibly be substituted by 1, 2, 3, 4, or 5 substituents, independently selected from the group consisting of F, Cl, Br, I, -CN, -CF₃, -SF₅, -OH, -O-CH₃, -O-C₂H₅, -NH₂, -NO₂, -O-CF₃, - S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-H, -C(=O)-CH₃, -C(=O)C₂H₅, -C(=O)-C(CH₃)₃, -C(=O)-NH₂, -C(=O)-NH-CH₃, -C(=O)-NH-C₂H₅, -C(=O)-N-(CH₃)₂, and -C(=O)-N-(C₂H₅)₂;
or is -(CH₂)-R³⁴, -(CH₂)-(CH₂)-R³⁴, or -(CH₂)-(CH₂)-(CH₂)-R³⁴.

8. The compounds according to one or several of the claims 1 to 7, **characterized in that**
R⁶ and R⁸, independently of each other are a residue selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n- heptadecyl, n-octadecyl, n-nonadecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl, and 3-butinyl, wherein the residue may possibly be substituted by 1, 2, 3, 4, or 5 substituents, independently selected from the group consisting of F, Cl, Br, I, -CN, -NO₂, -OH, -SH, and -NH₂;
is a residue selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, and indenyl, wherein the residue may possibly be substituted by 1, 2, 3, 4, or 5 substituents, independently selected from the group consisting of oxo (=O), thioxo (=S), F, Cl, Br, I, -CN, -CF₃, -SF₅, -OH, -O-CH₃, -O-C₂H₅, -O-C₁₋₅-alkyl, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, - C(=O)-O-CH₃, -C(=O)-O-C₂H₅, -C(=O)-O-C(CH₃)₃, -O-C(=O)-CH₃, -O-C(=O)C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may each be substituted by 1, 2, 3, 4, or 5 substituents, independently selected from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl;
is a residue selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl, and benzothiazolyl, wherein the residue may possibly be substituted by 1, 2, 3, 4, or 5 substituents, independently selected from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, - C(=O)-NH₂, -C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phenyl, -NH-S(=O)₂-CH₃, -NH-S(=O)₂-C₂H₅, - S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may each be substituted by 1, 2, 3, 4, or 5 substituents, independently selected from the group consisting of F, Cl, Br, -OH, - CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl;
is -(CHR³⁵)-C(=O)-R²⁴ or -(CHR³⁵)-(CH²)C(=O)-R²⁴;
is -(CHR³⁶)-C(=O)-O-R²⁵ or -(CHR³⁶)-(CH₂)-C(=O)-O-R²⁵;
or is -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹, or -(CR³⁷R³⁸)- (CHR³⁹)-(CHR⁴⁰)-N(C₂H₅)-R⁴¹.

9. The compounds according to one or several of the claims 1 to 8, **characterized in that**
R⁷ and R⁹ are each a hydrogen residue.

10. The compounds according to one or several of the claims 1 to 9, **characterized in that**
R¹⁰ to R²⁵, independently of each other, are each a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl, and 3-butinyl,
are a residue selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, and indenyl, wherein the residue may possibly be substituted by 1 or 2 substituents, independently selected from the group consisting of -C(=O)-OCH₃, -C(=O)-O-C₂H₅, -C(=O)-O-C(CH₃)₃, -C(=O)-CH₃, - C(=O)-C₂H₅, -C(=O)-C(CH₃)₃, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-phenyl, pyridinyl, phenyl, and benzyl, wherein the cyclic part of the residues pyridinyl, -S(=O)₂-phenyl, phenyl, and benzyl may be substituted by 1, 2, 3, 4, or 5 substituents, independently selected from the group consisting of F, Cl, Br, methyl, ethyl, n-propyl, isopropyl, n-butyl, sec-butyl, isobutyl, tert-butyl, -O-CH₃ and -O-C₂H₅;
are a residue selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, and pyrimidinyl, wherein the residue may possibly be substituted by 1, 2, 3, 4, or 5 substituents, independently selected from the group consisting of F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)- C₂H₅, -O-C(=O)-C(CH₃)₃, -N(CH₃)₂, - N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl, and phenyl,
or are -(CH₂)-R⁴² or-(CH₂)-(CH₂)-R⁴².

11. The compounds according to one or several of the claims 1 to 10, **characterized in that**
R²⁶, R²⁷, R²⁸, R³⁰, R³¹, R³², R³⁵, R³⁶, R³⁷, R³⁸, R³⁹, and R⁴⁰, independently of each other, are each
a hydrogen residue;
are a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl, and 3-butinyl;
or are a phenyl residue that may be substituted by 1, 2, 3, 4, or 5 substituents, independently selected from the group consisting of F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂. -N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, - O-benzyl, and phenyl.

12. The compounds according to one or several of the claims 1 to 11, **characterized in that**
R²⁹, R³³, and R⁴¹, independently of each other, are each a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl, and 3-butinyl, wherein the residue may possibly be substituted by 1, 2, 3, 4, or 5 substituents, independently selected from the group consisting of F, Cl, Br, I, -CN, -NO₂, -OH, -SH, and -NH₂;
are a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl, and (1,2,3,4)-tetrahydroquinazolinyl, wherein the residue may possibly each be substituted by 1, 2, 3, 4, or 5 substituents, independently selected from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)2-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -O-benzyl, phenyl, -(CH₂)-benzo[b]furanyl and benzyl may each be substituted by 1, 2, 3, 4, or 5 substituents, independently selected from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl;
is a residue selected from the group consisting of phenyl, naphthyl, (1,3)-benzodioxolyl, (1,4)-benzodioxanyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl, and benzothiazolyl, wherein the residue may possibly be substituted by 1, 2, 3, 4, or 5 substituents, independently selected from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)O-C₂H₅, -NH-C(=O)-O-C(CH₃)₃, -C(=O)-H, -C(=O)-CH₃, -C(=O)-C₂H₅, -C(=O)-C(CH₃)₃, - C(=O)-NH₂, -C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phenyl, -NH-S(=O)₂-CH₃, -NH-S(=O)₂-C₂H₅, - S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl, and benzyl, wherein the cyclic part of the residues of pyridinyl, cyclopentyl, cyclohexyl, pyridazinyl, -S(=O)₂-phenyl, -O-phenyl, -0-benzyl, phenyl, -(CH₂)-benzo[b]furanyl and benzyl may each be substituted by 1, 2, 3, 4, or 5 substituents, independently selected from the group consisting of F, Cl, Br, -OH, - CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl, and -O-benzyl.

13. The compounds according to one or several of the claims 1 to 12, **characterized in that**
R³⁴ and R⁴², independently of each other, are each a residue selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl and pyrimidinyl wherein the residue may possibly be substituted by 1, 2, 3, 4, or 5 substituents, independently selected from the group consisting of F, Cl, Br, I, -CN, -CF₃, -SF₅, -OH, -O-CH₃, -O-C₂H₅, -NH₂, -NO₂, -0-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

14. The compounds according to one or several of the claims 1 to 13, **characterized in that**
m equals 0.

15. The compounds according to one or several of the claims 1 to 14, **characterized in that** the compounds in the FLIPR assay, at a concentration of 10 µM, comprise an inhibition of the Ca²⁺ ion influx of the dorsal root ganglion of rats of at least 10%, preferably of at least 30%, particularly preferred of at least 50%, most preferably of at least 70%, further preferred of at least 90%, in comparison to a maximum achievable inhibition of the Ca²⁺ ion influx with Capsaicin at a concentration of 10 µM.

16. A method for producing substituted spiro-compounds of the general formula I according to one or several of the claims 1 to 15, **characterized in that** at least one compound of the general formula II 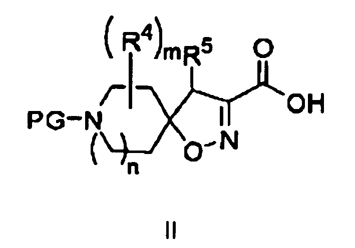 wherein R⁴, R⁵, m and n have the meaning according to one or several of the claims 1 to 15, and PG is a protective group, preferably for a benzyloxycarbonyl or tert-butyloxycarbonyl group, is converted in a reaction medium in the presence of at least one coupling reagent, possibly in the presence of at least one base with a compound of the general formula HNR²R³, wherein R² and R³ have the meaning according to one or several of the claims 1 to 15, to at least one compound of the general formula III, 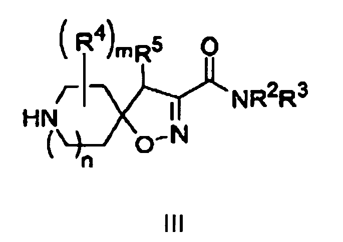 wherein R², R³, R⁴, R⁵, m, n, and PG have the meaning stated above, and the same is possibly purified and/or isolated,
and
at least one compound of the general formula III is converted in a reaction medium in the presence of at least one acid, preferably in the presence of at least one inorganic or organic acid, selected from the group consisting of hydrochloric acid, sulfuric acid, acetic acid and trifluoroacetic acid, or in the presence of hydrogen and a catalyst, preferably a catalyst based on palladium or platinum, to at least one compound of the general formula IV, 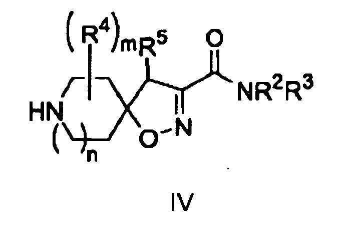 wherein R², R³, R⁴, R⁵, m and n have the meaning stated above, and the same is purified and/or isolated,
and
at least one compound of the general formula IV is converted in a reaction medium with at least one isocyanate of the general formula R⁶-N=C=O, wherein R⁶ has the meaning according to one or several of the claims 1 to 15, possibly in the presence of at least one base, preferably in the presence of at least one base selected from the group consisting of triethylamine, 4,4-dimethylaminopyridine, and diisopropylethylamine, to at least one compound of the general formula I, wherein R², R³, R⁴, R⁵, m and n have the meaning stated above and R¹ is -C(=O)-NR⁶R⁷, wherein R⁶ has the meaning stated above and R⁷ is a hydrogen residue, and the same is possibly purified and/or isolated,
or
at least one compound of the general formula IV is converted in a reaction medium with at least one isothiocyanate of the general formula S=C=N-R⁸, wherein R⁸ has the meaning according to one or several of the claims 1 to 15, possibly in the presence of at least one base, preferably in the presence of at least one base selected from the group consisting of triethylamine, 4,4-dimethylaminopyridine and diisopropylethylamine, to at least one compound of the general formula I, wherein R², R³, R⁴, R⁵, m and n have the meaning stated above, and R¹ is -C(=S)-N-R⁸R⁹, wherein R⁸ has the meaning stated above and R⁹ is a hydrogen residue, and the same is possibly purified and/or isolated,
and possibly at least one compound of the general formula I, wherein R², R³, R⁴, R⁵, m and n have the meaning stated above and R¹ is -C(=O)-NR⁶R⁷ or -C(=S)-N-R⁸R⁹, wherein R⁷ and R⁹ are each a hydrogen residue, is converted in a reaction medium in the presence of at least one base, preferably in the presence of at least one metal hydride salt or of a metal alcoholate salt, particularly preferred in the presence of a metal hydride salt or of a metal alcoholate salt selected from the group consisting of sodium hydride, potassium hydride, potassium- tert-butanolate, sodium-tert-butanolate, potassium methanolate, sodium methanolate, sodium ethanolate, and potassium ethanolate, with at least one compound of the general formula LG-R⁷ or of the general formula LG-R⁹, wherein LG is a leaving group, preferably for a halogen atom, particularly preferred for a chlorine atom, and R⁷ and R⁹ have the meaning stated above, with the exception of hydrogen, to at least one compound of the general formula I, wherein R¹ to R⁵, m and n have the meaning stated above, and the same is possibly purified and/or isolated,
or
at least one compound of the general formula IV is converted in a reaction medium in the presence of at least one base, preferably in the presence of at least one metal hydride salt, particularly preferred in the presence of sodium and/or potassium hydride, with at least one compound of the general formula LG-R¹, wherein R¹ has the meaning according to one or several of the claims 1 to 15, with the exception of -C(=O)-NR⁶R⁷ and -C(=S)-NR⁸R⁹, and LG is a leaving group, preferably a halogen atom, particularly preferably a chlorine atom, to at least one compound of the general formula I, wherein R¹ to R⁵, m and n have the meaning stated above, and the same is possibly purified and/or isolated,
or
at least one compound of the general formula IV is converted in a reaction medium in the presence of at least one reduction agent with at least one compound of the general formula R¹-C(=O)-H, wherein R¹ has the meaning according to one or several of the claims 1 to 15, with the exception of -C(=O)-NR⁶R⁷ and -C(=S)-NR⁸R⁹, to at least one compound of the general formula I, wherein R¹ to R⁵, m and n have the meaning stated above, and the same is possibly purified and/or isolated.

17. A pharmaceutical comprising at least one compound according to one or several of the claims 1 to 15 and possibly one or several physiologically compatible excipients.

18. The pharmaceutical according to claim 17 for the prophylaxis and/or treatment of pain, preferably of pain selected from the group consisting of acute pain, chronic pain, neuropathic pain, and visceral pain.

19. The pharmaceutical according to claim 17 for the treatment and/or prophylaxis of one or more diseases selected from the group consisting of pain, preferably pain selected from the group consisting of acute pain, chronic pain, neuropathic pain and visceral pain; joint pain, migraine; depression; neuropathy; nerve injury; neurodegenerative diseases, preferably selected from the group consisting of multiple sclerosis, Alzheimer's disease, Parkinson's disease and Huntington's disease; cognitive dysfunctions, preferably cognitive deficiencies, particularly preferred disturbances of memory; epilepsy; respiratory disorders, preferably selected from the group consisting of asthma and pneumonia; cough; aconuresis; overactive bladder (OAB); stomach ulcers; irritable bowel syndrome; stroke; eye irritations; skin irritations; neurotic skin disorders; inflammation, preferably inflammation of the intestines; diarrhea; pruritus; ingestion disorders, preferably selected from the group consisting of bulimia, cachexia, anorexia and obesity; drug dependency; drug abuse; withdrawal symptoms with drug dependency; development of tolerances to pharmaceuticals, preferably to natural or synthetic opioids; substance dependency; substance abuse, withdrawal symptoms with substance dependency; alcohol dependency; alcohol abuse and withdrawal symptoms with alcohol dependency; for dieresis; for antinatriuresis; for influencing the cardiovascular system; for increasing vigilance; for increasing libido; for the modulation of movement and activity; for anxiolysis; for local anesthesia, and/or for inhibiting undesired side effects, preferably selected from the group consisting of hyperthermia, high blood pressure, bronchial constriction caused by the administration of Vanilloid receptor 1 (VR1/TRPV1-receptors) agonists, preferably selected from the group consisting of Capsaicin, Resiniferatoxin, Olvanil, Arvanil, SDZ-249665, SDZ-249482, Nuvanil and Capsavanil (DA-5018).

20. Use of at least one compound according to one or several of the claims 1 to 15 for producing a pharmaceutical for the prophylaxis and/or treatment of pain, preferably of pain selected from the group consisting of acute pain, chronic pain and neuropathic pain.

21. The use of at least one compound according to one or several of the claims 1 to 15 for producing a pharmaceutical for the prophylaxis and/or treatment of one or more diseases selected from the group consisting of pain, preferably of pain selected from the group consisting of acute pain, chronic pain, neuropathic pain and visceral pain; joint pain; migraine; depression; neuropathy; nerve injury; neurodegenerative diseases, preferably selected from the group consisting of multiple sclerosis, Alzheimer's disease, Parkinson's disease and Huntington's disease; cognitive dysfunctions, preferably cognitive deficiencies, particularly preferred disturbances of memory; epilepsy; respiratory disorders, preferably selected from the group consisting of asthma and pneumonia; cough; aconuresis; overactive bladder (OAB); stomach ulcers; irritable bowel syndrome; stroke; eye irritations, skin irritations; neurotic skin disorders; inflammation, preferably inflammation of the intestines; diarrhea; pruritus; ingestion disorders, preferably selected from the group consisting of bulimia, cachexia, anorexia and obesity; drug dependency; drug abuse; withdrawal symptoms with drug dependency; development of tolerances to pharmaceuticals, preferably to natural or synthetic opioids; substance dependency; substance abuse; withdrawal symptoms with substance dependency; alcohol dependency; alcohol abuse and withdrawal symptoms with alcohol dependency; for dieresis; for antinatriuresis; for influencing the cardiovascular system; for increasing vigilance; for increasing libido; for the modulation of movement and activity; for anxiolysis; for local anesthesia, and/or for inhibiting undesired side effects, preferably selected from the group consisting of hyperthermia, high blood pressure, bronchial constriction caused by the administration of Vanilloid receptor 1 (VR1/TRPV1-receptors) agonists, preferably selected from the group consisting of Capsaicin, Resiniferatoxin, Olvanil, Arvanil, SDZ-249665, SDZ-249482, Nuvanil and Capsavanil (DA-5018).

## Revendications

1. Composés spiro substitués de formule générale I, 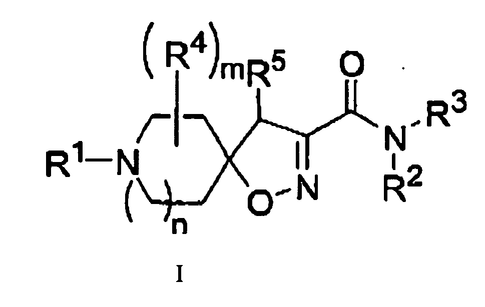 dans laquelle
m est égal à 0, 1, 2, 3 ou 4,
n est égal à 0,
R¹ représente un radical aliphatique en C₁ à C₁₀ linéaire ou ramifié, saturé ou insaturé, substitué le cas échéant ;
un radical cycloaliphatique à 3, 4, 5, 6, 7, 8 ou 9 éléments, insaturé ou saturé, substitué le cas échéant, qui peut être condensé avec un système cyclique mono- ou polycyclique saturé, insaturé ou aromatique, substitué le cas échéant ;
un radical aryle ou hétéroaryle, substitué le cas échéant, de 5 à 14 éléments, qui peut être condensé avec un système cyclique mono- ou polycyclique saturé ou insaturé, substitué le cas échéant,
un groupe -C(=S)-NR⁸R⁹, ou
-(CHR²⁶)-X_{q}(CHR²⁷)ᵣ-Yₛ-(CHR²⁸)ₜ-Zᵤ-R²⁹ avec q = 0 ou 1, r = 0 ou 1, s = 0 ou 1, t = 0 ou 1, u = 0 ou 1, dans lequel X, Y et Z, indépendamment les uns des autres, signifient respectivement O, S, NH, N(CH₃), N(C₂H₅) ou N[CH(CH₃)₂], ou
dans la mesure où m est différent de 0 et/ou n est différent de 1 et/ou R⁵ est différent de H, il peut signifier en plus un groupe -C(=O)-NR⁶R⁷,
R² représente un radical aliphatique en C₁ à C₁₀ linéaire ou ramifié, saturé ou insaturé, substitué le cas échéant ;
un radical cycloaliphatique à 3, 4, 5, 6, 7, 8 ou 9 éléments, insaturé ou saturé, substitué le cas échéant, qui peut être condensé avec un système cyclique mono- ou polycyclique saturé, insaturé ou aromatique, substitué le cas échéant
un radical aryle ou hétéroaryle substitué le cas échéant de 5 à 14 éléments, qui peut être condensé avec un système cyclique mono- ou polycyclique saturé ou insaturé, substitué le cas échéant ;
ou -(CHR³⁰)-Xᵥ-(CHR³¹)_{w}-Yₓ-(CHR³²)_{y}-Z_{z}-R³³ avec v = 0 ou 1, w = 0 ou 1, x = 0 ou 1, y = 0 ou 1, z = 0 ou 1, dans lequel X, Y et Z, indépendamment les uns des autres, signifient respectivement O, S, NH, N(CH₃), N(C₂H₅) ou N[CH(CH₃)₂] ;
R³ signifie un radical hydrogène ;
un radical cycloaliphatique linéaire ou ramifié, saturé ou insaturé, substitué le cas échéant ;
un radical cycloaliphatique de 3, 4, 5, 6, 7, 8 ou 9 éléments, insaturé ou saturé, substitué le cas échéant, qui peut être condensé avec un système cyclique mono- ou polycyclique saturé, insaturé ou aromatique, substitué le cas échéant ;
un radical aryle ou hétéroaryle substitué le cas échéant de 5 à 14 éléments, qui peut être condensé avec un système cyclique mono- ou polycyclique saturé ou insaturé, substitué le cas échéant,
ou -(CHR³⁰)-Xᵥ-(CHR³¹)_{w}-Yₓ-(CHR³²)_{y}-Z_{z}-R³³ avec v = 0 ou 1, w = 0 ou 1, x = 0 ou 1, y = 0 ou 1, z = 0 ou 1, dans lequel X, Y et Z, indépendamment les uns des autres, signifient respectivement O, S, NH, N(CH₃), N(C₂H₅) ou N[CH(CH₃)₂] ;
ou
R² et R³ forment conjointement avec l'atome d'azote qui les relie comme élément du cycle un radical hétérocycloaliphatique saturé ou insaturé, substitué le cas échéant à 4, 5, 6, 7, 8 ou 9 éléments, qui peut être condensé avec un système cyclique mono- ou polycyclique saturé, insaturé ou aromatique, substitué le cas échéant,
R⁴ représente un radical halogène, un groupe nitryle, un groupe cyano, un groupe amino, un groupe hydroxyle, un groupe thiol, un groupe carboxyle, un groupe formyle, un groupe -NH-C(=O)-H, un groupe oxo (=O), un groupe -NH-R¹⁰, un groupe N, un groupe -C(=O)-R¹³, un groupe -C(=O)-O-R¹⁴, un groupe -O-C(=O)-R¹⁵, un groupe -NH-C(=O)-R¹⁶, un groupe -NR¹⁷-C(=O)-R¹⁸, un groupe -C(=O)-NH₂, un groupe -C(=O)-NH-R¹⁹, un groupe -C(=O)-NR²⁰R²¹, un groupe -O-R²², un groupe -S-R²³, ou un radical aliphatique en C₁ à C₁₀, linéaire ou ramifié, saturé ou insaturé, substitué le cas échéant ;
R⁵ représente un radical hydrogène, un radical halogène, un groupe nitryle, un groupe cyano, un groupe amino, un groupe hydroxyle, un groupe thiol, un groupe carboxyle, un groupe formyle, un groupe -NH-C(=O)-H, un groupe oxo (=O), un groupe -NH-R¹⁰, un groupe -NR¹¹ R¹², un groupe -C(=O)-R¹³, un groupe -C=O)-O-R¹⁴, un groupe -O-C(=O)-R¹⁵, un groupe -NH-C(=O)-R¹⁶, un groupe -NR¹⁷-C(=O)-R¹⁸, un groupe -C(=O)-NH₂, un groupe -C(=O)-NH-R¹⁹, un groupe -C(=O)-NR²⁰R²¹, un groupe -O-R²², un groupe -S-R²³, un radical aliphatique en C₁ à C₁₀ linéaire ou ramifié, saturé ou insaturé, substitué le cas échéant ;
un radical aryle ou hétéroaryle de 5 à 14 éléments, substitué le cas échéant
ou -(CH₂)ₐₐ-R³⁴ avec aa = 1, 2, 3 ou 4 ;
R⁶ et R⁸ représentent, indépendamment l'un de l'autre, respectivement
un radical aliphatiques en C₁ à C₂₀ linéaire ou ramifié, saturé ou insaturé, substitué le cas échéant ;
un radical cycloaliphatique insaturé ou saturé, substitué le cas échéant à 3, 4, 5, 6, 7, 8, 9, 10, 11 ou 12 éléments, qui peut être condensé avec un système cyclique mono- ou polycyclique saturé, insaturé ou aromatique, substitué le cas échéant et/ou ponté par un ou deux groupes alkylènes en C₁ à C₅ linéaires ou ramifiés, substitués le cas échéant ;
un radical aryle ou hétéroaryle substitué le cas échéant de 5 à 14 éléments, qui peut être condensé avec un système cyclique mono- ou polycyclique saturé ou insaturé, substitué le cas échéant ;
-(CHR³⁵)-(CH₂)_{bb}-(CH₂)_{cc}-C(O)-R²⁴ avec bb = 0 ou 1 et cc = 0 ou 1 ;
-(CHR³⁶)-(CH₂)_{dd}-(CH₂)ₑₑ-C(=O)-O-R²⁵ avec dd = 0 ou 1 et ee = 0 ou 1 ;
ou -(CR³⁷R³⁸)-X_{ff}(CHR³⁹)_{gg}-Yₕₕ-(CHR⁴⁰)ⱼⱼ-Zₖₖ-R⁴¹ avec ff = 0 ou 1, gg = 0 ou 1, hh = 0 ou 1, jj = 0 ou 1, kk = 0 ou 1, dans lequel X, Y et Z, indépendamment les uns des autres, représentent respectivement O, S, NH, N(CH₃), N(C₂H₅) ou N[CH(CH₃)₂] ;
R⁷ et R⁹ représentent, indépendamment l'un de l'autre, respectivement
un radical hydrogène ;
un radical aliphatique en C₁ à C₂₀ linéaire ou ramifié, saturé ou insaturé, substitué le cas échéant ;
un radical cycloaliphatique insaturé ou saturé, substitué le cas échéant à 3, 4, 5, 6, 7, 8, 9, 10, 11 ou 12 éléments, qui peut être condensé avec un système cyclique mono- ou polycyclique saturé, insaturé ou aromatique, substitué le cas échéant et/ou ponté par un ou deux groupes alkylènes en C₁ à C₅ linéaires ou ramifiés, substitués le cas échéant ;
un radical aryle ou hétéroaryle substitué le cas échéant de 5 à 14 éléments, qui peut être condensé avec un système cyclique mono- ou polycyclique saturé ou insaturé, substitué le cas échéant ;
-(CHR³⁵)-(CH₂)_{bb}-(CH₂)_{cc}-C(=O)-R²⁴ avec bb = 0 ou 1 et cc = 0 ou 1 ;
-(CHR³⁶)-(CH₂)_{dd}-(CH₂)ₑₑ, -C(=O)-O-R²⁵ avec dd = 0 ou 1 et ee 0 ou 1 ;
ou -(CR³⁷R³⁸)-X_{ff}(CHR³⁹)_{gg}-Yₕₕ-(CHR⁴⁰)ⱼⱼ-Zₖₖ-R⁴¹ avec ff= 0 ou 1, gg = 0 ou 1, hh = 0 ou 1, jj = 0 ou 1, kk = 0 ou 1, dans lequel X, Y et Z, indépendamment les uns des autres, représentent respectivement O, S, NH, N(CH₃), N(C₂H₅) ou N[CH(CH₃)₂] ;
R¹⁰ à R²⁵ représentent, indépendamment l'un de l'autre, respectivement
un radical aliphatique en C₁ à C₁₀ linéaire ou ramifié, saturé ou insaturé, substitué le cas échéant ;
un radical cycloaliphatique à 3, 4, 5, 6, 7, 8 ou 9 éléments, insaturé ou saturé, substitué le cas échéant, qui peut être condensé avec un système cyclique mono- ou polycyclique saturé, insaturé ou aromatique, substitué le cas échéant ;
un radical aryle ou hétéroaryle substitué le cas échéant, de 5 à 14 éléments, qui peut être condensé avec un système cyclique mono- ou polycyclique saturé ou insaturé, substitué le cas échéant,
ou -(CH₂)ₘₘ-R⁴² avec mm égal à 1, 2 ou 3 ;
R²⁶, R²⁷ , R²⁸, R³⁰, R³¹, R³², R³⁵, R³⁶, R³⁷, R³⁸, R³⁸ et R⁴⁰ représentent, indépendamment les uns des autres, respectivement
un radical hydrogène
un radical aliphatique en C₁ à C₁₀ linéaire ou ramifié, saturé ou insaturé, substitué le cas échéant,
ou un radical aryle ou hétéroaryle substitué le cas échéant de 5 à 14 éléments, qui peut être condensé avec un système cyclique mono- ou polycyclique saturé ou insaturé, substitué le cas échéant ;
R²⁹, R³³ et R⁴¹, représentent, indépendamment les uns des autres, respectivement
un radical aliphatique en C₁ à C₁₀ linéaire ou ramifié, saturé ou insaturé, substitué le cas échéant,
un radical cycloaliphatique insaturé ou saturé, substitué le cas échéant, à 3, 4, 5, 6, 7, 8 ou 9 éléments, qui peut être ponté par 1, 2, 3, 4 ou 5 groupes alkylènes en C₁ à C₅ linéaires ou ramifiés, substitués le cas échéant et/ou qui peut être condensé avec un système cyclique mono- ou polycyclique saturé, insaturé ou aromatique, substitué le cas échéant ;
un radical aryle ou hétéroaryle substitué le cas échéant, à 5 à 14 éléments, qui peut être condensé avec un système cyclique mono- ou polycyclique saturé ou insaturé, substitué le cas échéant ;
et
R³⁴ et R⁴² représentent, indépendamment l'un de l'autre, respectivement
un radical aryle ou hétéroaryle substitué le cas échéant, de 5 à 14 éléments ;
sachant que
les radicaux aliphatiques en C₁ à C₁₀ et les radicaux aliphatiques en C₁ à C₂₀ cités ci-avant peuvent chacun être substitués le cas échéant par 1, 2, 3, 4, 5, 6, 7, 8 ou 9 substituants choisis indépendamment les uns des autres parmi le groupe constitué de F, Cl, Br, I, -CN, -NO₂, -OH, -SH et -NH₂ ;
les radicaux cycloaliphatiques et hétérocycloaliphatiques cités ci-avant peuvent être substitués respectivement le cas échéant par 1, 2, 3, 4 ou 5 substituants choisis indépendamment les uns des autres parmi le groupe constitué de oxo (=O), thioxo (=S), F, Cl, Br, I, -CN, -CF₃, -SF₅, -OH, -O-(alkyle en C₁ à C₅), -NH₂, -NO₂, -O-CF₃, -S-CF₃, -SH, -S-(alkyle en C₁ à C₅), -(alkyle en C₁ à C₅), -C(=O)-OH, -O-C(=O)-(alkyle en C₁ à C₅), -NH-(alkyle en C₁ à C₅), -N(alkyle en C₁ à C₅)₂, -NH-C(=O)-O-(alkyle en C₁ à C₅), -C(=O)-H, -C(=O)-(alkyle en C₁ à C₅), -C(=O)-NH₂, -C(=O)-NH-(alkyle en C₁ à C₅), C(=O)-N-(alkyle en C₁ à C₅)₂, -S(=O)₂-(alkyle en C₁ à C₅), -S(=O)₂-phényle, -NH-S(=O)₂-(alkyle en C₁ à C₅), -S(=O)₂-NH-(alkyle en C₁ à C₅), cyclohexyle, cyclopentyle, pyridinyle, pyridazinyle, -(CH₂)-benzo[b]furanyle, -O-phényle, -O-benzyle, phényle et benzyle, sachant qu'à chaque fois la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, -(alkyle en C₁ à C₅), -O-(alkyle en C₁ à C₅), -O-CF₃, -S-CF₃, phényle et -O-benzyle,
et les radicaux cycloaliphatiques cités ci-avant peuvent comporter respectivement le cas échéant comme éléments du cycle 1, 2, 3, 4 ou 5 hétéroatomes choisis indépendamment les uns des autres parmi le groupe constitué de l'oxygène, de l'azote et du soufre ;
les radicaux hétérocycloaliphatiques cités ci-avant peuvent comporter respectivement le cas échéant comme éléments du cycle 1, 2 ou 3 hétéroatomes supplémentaires choisis indépendamment les uns des autres parmi le groupe constitué de l'oxygène, de l'azote et du soufre ;
les cycles des systèmes cycliques mono- ou polycycliques cités ci-avant peuvent être substitués le cas échéant chacun par 1, 2, 3, 4 ou 5 substituants choisis indépendamment les uns des autres parmi le groupe constitué de oxo (=O), thioxo (=S), F, Cl, Br, I, -CN, -CF₃, -SF₅, -OH, -O-(alkyle en C₁ à C₅), -NH₂, -NO₂, -O-CF₃, -S-CF₃, -SH, -S-(alkyle en C₁ à C₅), -(alkyle en C₁ à C₅), -O-C(=O)-(alkyle en C₁ à C₅), -NH-(alkyle en C₁ à C₅), -N(alkyle en C₁ à C₅)₂, -NH-C(=O)-O-(alkyle en C₁ à C₅), -C(=O)-H, -C(=O)-(alkyle en C₁ à C₅), -C(=O)-NH₂, -C(=O)-NH-(alkyle en C₁ à C₅), -C(=O)-N-(alkyle en C₁ à C₅)₂, -S(=O)₂-(alkyle en C₁ à C₅), -S(=O)₂-phényle, -NH-S(=O)₂-(alkyle en C₁ à C₅), -S(=O)₂-NH-(alkyle en C₁ à C₅), cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, -(alkyle en C₁ à C₅), -O-(alkyle en C₁ à C₅), -O-CF₃, -S-CF₃, phényle et -O-benzyle,
et les cycles des systèmes cycliques mono- ou polycycliques cités ci-avant sont respectivement à 5, 6 ou 7 éléments et peuvent comporter respectivement le cas échéant comme éléments du cycle 1, 2, 3, 4 ou 5 hétéroatomes qui, indépendamment les uns des autres, sont choisis parmi le groupe constitué de l'oxygène, de l'azote et du soufre ;
et les radicaux aryles ou hétéroaryles cités ci-avant peuvent être substitués le cas échéant chacun par 1, 2, 3, 4 ou 5 substituants choisis indépendamment les uns des autres parmi le groupe constitué de F, Cl, Br, I, -CN, -CF₃, -SF₅, -OH, -O-(alkyle en C₁ à C₅), -NH₂, -NO₂, -O-CF₃, -S-CF₃, -SH, -S-(alkyle en C₁ à C₅), -(alkyle en C₁ à C₅), -C(=O)-OH, -C(=O)-O-(alkyle en C₁ à C₅), -O-C(=O)-(alkyle en C₁ à C₅), -N(alkyle en C₁ à C₅)₂, -NH-C(=O)-O-(alkyle en C₁ à C₅), -C(=O)-H, -C(=O)-(alkyle en C₁ à C₅), -C(=O)-NH₂, -C(=O)-NH-(alkyle en C₁ à C₅), C(=O)-N-(alkyle en C₁ à C₅)₂, -S(=O)₂-(alkyle en C₁ à C₅), -S(=O)₂-phényle, -NH-S(=O)₂-(alkyle en C₁ à C₅), -S(=O)₂-NH-(alkyle en C₁ à C₅), cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, -(alkyle en C₁ à C₅), -O-(alkyle en C₁ à C₅), -O-CF₃, -S-CF₃, phényle et -O-benzyle,
les radicaux hétéroaryles cités ci-avant peuvent comporter respectivement le cas échéant comme éléments du cycle 1, 2, 3, 4 ou 5 hétéroatomes choisis indépendamment les uns des autres parmi le groupe constitué de l'oxygène, de l'azote et du soufre ;
et
les groupes alkylènes en C₁ à C₅ cités ci-avant peuvent être substitués respectivement le cas échéant par 1, 2, 3, 4 ou 5 substituants choisis indépendamment les uns des autres parmi le groupe constitué de F, Cl, Br, -OH, -SH, -NH₂, -CN et NO₂ ;
respectivement le cas échéant sous la forme d'un de leur stéréo-isomères purs, en particulier énantiomères ou dia-stéréo-isomères, de leurs racemates ou sous la forme d'un mélange de stéréo-isomères, en particulier des énantiomères et/ou des dia-stéréo-isomères, dans un rapport de mélange quelconque, ou respectivement sous la forme de sels correspondants, ou respectivement sous la forme de solvates correspondants.

2. Composés selon la revendication 1, **caractérisés en ce que**
R¹ représente un radical choisi parmi le groupe constitué du méthyle, de l'éthyle, du n-propyle, de l'isopropyle, du n-butyle, du sec-butyle, de l'isobutyle, du tert-butyle, du n-pentyle, du sec-pentyle, de -(CH₂)-(CH₂)-(C(CH₃)₃), du n-hexyle, du n-heptyle, du n-octyle, du -(CH₂)-(CH)(C₂H₅)-(CH₂)-(CH₂)-(CH₂)-(CH₃), du vinyle, du 1-propényle, du 2-propényle, du 1-butényle, du 2-butényle, du 3-butényle, de l'éthinyle, du 1-propinyle, du 2-propinyle, du 1-butinyle, du 2-butinyle et du 3-butinyle, sachant que le radical peut être substitué le cas échéant respectivement par 1, 2, 3, 4 ou 5 substituants choisis indépendamment les uns des autres parmi le groupe constitué de F, Cl, Br, I, -CN, -NO₂, -OH, -SH et -NH₂ ;
un radical choisi parmi le groupe constitué du cyclopropyle, du cyclobutyle, du cyclopentyle, du cyclohexyle, du cycloheptyle, du cyclopentényle, du cyclohexényle, du cyclohéptenyle, de l'imidazolidinyle, du tétrahydrofuranyle, du tétrahydrothiophényle, du pyrrolidinyle, du pipéridinyle, du morpholinyle, du pipérazinyle, du thiomorpholinyle, du tétrahydropyranyle, de l'azépanyle, du diazépanyle, du dithiolanyle, de l'indanyle, de l'indényle, du (1, 4)-benzodioxanyle, du (1, 2, 3, 4)-tétrahydronaphthyle, du (1, 2, 3, 4)-tétrahydroquinolinyle et du (1, 2, 3, 4)-tétrahydroquinazolinyle, sachant que le radical peut être substitué le cas échéant respectivement par 1, 2, 3, 4 ou 5 substituants choisis indépendamment les uns des autres parmi le groupe constitué de l'oxo (=O), du thioxo (=S), de F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-Propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(-O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un radical choisi parmi le groupe constitué de phényle, naphthyle, (1,3)-benzodioxolyle, (1,4)-benzodioxanyle, thiophényle, furanyle, pyrrolyle, pyrazolyle, pyranyle, triazolyle, pyridinyle, imidazolyle, indolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, sachant que le radical peut être substitué le cas échéant respectivement par 1, 2, 3, 4 ou 5 substituants choisis indépendamment les uns des autres parmi le groupe constitué de F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un groupe -C(=S)-NR⁸R⁹ ; ou
-(CHR²⁶)-R²⁹ ; -(CHR²⁶)-(CHR²⁷)-R²⁹ ; -(CHR²⁶)-(CHR²⁷)-O-R¹⁹; -(CHR²⁶)-(CHR²⁷)-(CHR²⁸)-R²⁹; -(CHR²⁶)-(CHR²⁷)-S-(CHR²⁸)-R²⁹ ; -(CHR²⁶)-(CHR²⁷)-(CHR²⁸)-N(CH₃)-R²⁹, ou
dans la mesure où m est différent de 0 et/ou n est différent de 1 et/ou R⁵ est différent de H, en plus un groupe -C(=O)-NR⁶R⁷.

3. Composés selon la revendication 1 à 2, **caractérisés en ce que**
R² représente un radical choisi parmi le groupe constitué de méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthinyle, 1-propinyle, 2-propinyle, 1-butinyle, 2-butinyle et 3-butinyle, sachant que le radical peut être substitué le cas échéant respectivement par 1, 2, 3, 4 ou 5 substituants choisis indépendamment les uns des autres parmi le groupe constitué de F, Cl, Br, I, -CN, -NO₂, -OH, -SH et -NH₂;
un radical choisi parmi le groupe constitué du cyclopropyle, du cyclobutyle, du cyclopentyle, du cyclohexyle, du cycloheptyle, du cyclopentényle, du cyclohexényle, du cycloheptényle, du tétrahydrofuranyle, du tétrahydrothiophényle, du pyrrolidinyle, du pipéridinyle, du morpholinyle, du pipérazinyle, du thiomorpholinyle, du tétrahydropyranyle, de l'azépanyle, du diazépanyle, du dithiolanyle, de l'indanyle, de l'indényle, du (1, 4)-benzodioxanyle, du (1, 2, 3, 4)-tétrahydronaphthyle, du (1, 2, 3, 4)-tétrahydroquinolinyle et du (1, 2, 3, 4)-tétrahydroquinazolinyle, sachant que le radical peut être substitué le cas échéant respectivement par 1, 2, 3, 4 ou 5 substituants choisis indépendamment les uns des autres parmi le groupe constitué de oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-Butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un radical choisi parmi le groupe constitué de phényle, naphthyle, (1, 3)-benzodioxolyle, (1, 4)-benzodioxanyle, thiophényle, furanyle, pyrrolyle, pyrazolyle, pyranyle, triazolyle, pyridinyle, imidazolyle, indolyle, iso-indolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, sachant que le radical peut être substitué le cas échéant respectivement par 1, 2, 3, 4 ou 5 substituants choisis indépendamment les uns des autres parmi le groupe constitué de F, Cl, Br, I, -CN, -CF₃, -SF₅, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-OC(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, CI, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
ou -(CHR³⁰)-R³³; -(CHR³⁰)-(CHR³¹)-R³³; -(CHR³⁰)-(CHR³¹)-O-R³³; -(CHR³⁰-(CHR³¹)-(CHR³²)-R³³; -(CHR³⁰)-(CHR³¹)-S-(CHR³²)-R³³; -(CHR³⁰-(CHR³¹)-(CHR³²)-N(CH₃)-R³³.

4. Composés selon une ou plusieurs des revendications 1 à 3, **caractérisés en ce que**
R³ représente un radical hydrogène.

5. Composés selon une ou plusieurs des revendications 1 à 4, **caractérisés en ce que**
R² et R³ forment conjointement avec l'atome d'azote qui les relie comme élément du cycle un radical choisi parmi le groupe constitué de pyrrolidinyle, pipéridinyle, (1, 3, 4, 5)-tétrahydropyrido[4, 3-b]indolyle, (3, 4)-dihydro-1H-isoquinolinyle, (1, 3, 4, 9)-tétrahydro-[b]-carbolinyle, imidazolidinyle, (1, 3)-thiazolidinyle, pipérazinyle, morpholinyle, azépanyle, diazépanyle et thiomorpholinyle, sachant que le radical peut être substitué respectivement par 1, 2, 3, 4 ou 5 substituants choisis indépendamment les uns des autres parmi le groupe constitué de oxo (=O), thioxo (=S), F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(-O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle.

6. Composés selon une ou plusieurs des revendications 1 à 5, **caractérisés en ce que**
R⁴ représente un radical choisi parmi le groupe constitué de méthyle, éthyle, n-propyle, isopropyle, n-butyle, sec-butyle, isobutyle, tert-butyle, n-pentyle, sec-pentyle, n-hexyle et n-heptyle, sachant que le radical peut être substitué le cas échéant respectivement par 1, 2, 3, 4 ou 5 substituants choisis indépendamment les uns des autres parmi le groupe constitué de F, Cl, Br, I, -CN, -NO₂, -OH, -SH et -NH₂.

7. Composés selon une ou plusieurs des revendications 1 à 6, **caractérisés en ce que**
R⁵ représente un radical hydrogène,
un radical choisi parmi le groupe constitué de méthyle, éthyle, n-propyle, isopropyle, n-butyle, sec-butyle, isobutyle, tert-butyle, n-pentyle, sec-pentyle, n-hexyle et n-heptyle, sachant que le radical peut être substitué le cas échéant respectivement par 1, 2, 3, 4 ou 5 substituants choisis indépendamment les uns des autres parmi le groupe constitué de F, Cl, Br, I, -CN, -NO₂, -OH, -SH et -NH₂ ;
un radical choisi parmi le groupe constitué de phényle, naphthyle, thiophényle, furanyle, pyrrolyle, pyrazolyle, pyranyle, triazolyle, pyridinyle, imidazolyle, indolyle, iso-indolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, sachant que le radical peut être substitué le cas échéant respectivement par 1, 2, 3, 4 ou 5 substituants choisis indépendamment les uns des autres parmi le groupe constitué de F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂ et -C(=O)-N-(C₂H₅)₂ ;
ou -(CH₂)-R³⁴, -(CH₂)-(CH₂)-R³⁴ ou -(CH₂)-(CH₂)-(CH₂)-R³⁴.

8. Composés selon une ou plusieurs des revendications 1 à 7, **caractérisés en ce que**
R⁶ et R⁸ représentent, indépendamment l'un de l'autre, respectivement un radical choisi parmi le groupe constitué de méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthinyle, 1-propinyle, 2-propinyle, 1-butinyle, 2-butinyle et 3-butinyle, sachant que le radical peut être substitué le cas échéant respectivement par 1, 2, 3, 4 ou 5 substituants choisis indépendamment les uns des autres parmi le groupe constitué de F, Cl, Br, I, -CN, -NO₂, -OH, -SH et -NH₂ ;
un radical choisi parmi le groupe constitué de cyclopropyle, cyclobutyle, cyclopentyle, cyclohexyle, cycloheptyle, cyclo-octyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, sachant que le radical peut être substitué le cas échéant respectivement par 1, 2, 3, 4 ou 5 substituants choisis indépendamment les uns des autres parmi le groupe constitué de oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un radical choisi parmi le groupe constitué de phényle, naphthyle, (1, 3)-benzodioxolyle, (1, 4)-benzodioxanyle, thiophényle, furanyle, pyrrolyle, pyrazolyle, pyranyle, triazolyle, pyridinyle, imidazolyle, indolyle, iso-indolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, sachant que le radical peut être substitué le cas échéant respectivement par 1, 2, 3, 4 ou 5 substituants choisis indépendamment les uns des autres parmi le groupe constitué de F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=_{O})-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
représentent -(CHR³⁵)-C(=O)-R²⁴ ou -(CHR³⁵)-(CH₂)-C(=O)-R²⁴ ;
représentent -(CHR³⁶)-C(=O)-O-R²⁵ ou -(CHR³⁶)-(CH₂)-C(=O)-O-R²⁵ ;
ou représentent -(CR³⁷R³⁸)-R⁴¹, -(CR³⁷R³⁸)-(CHR³⁹)-R⁴¹, -(CR³⁷R³⁸)-(CHR³⁹)-O-R⁴¹, -(CR³⁷R³⁸)-(CHR³⁹)-(CHR⁴⁰)-R⁴¹, -(CR³⁷R³⁸)-(CHR³⁹)-(CHR⁴⁰)-O-R⁴¹, -(CR³⁷R³⁸)-(CHR³⁹)-(CHR⁴⁰)-N(CH₃)-R⁴¹ ou -(CR³⁷R³⁹)-(CHR³⁹)-(CHR⁴⁰)-N(C₂H₅)-R⁴¹.

9. Composés selon une ou plusieurs des revendications 1 à 8, **caractérisés en ce que**
R⁷ et R⁹ représentent chacun un radical hydrogène.

10. Composés selon une ou plusieurs des revendications 1 à 9, **caractérisés en ce que**
R¹⁰ à R²⁵ représentent indépendamment l'un de l'autre respectivement un radical choisi parmi le groupe constitué de méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthinyle, 1-propinyle, 2-propinyle, 1-butinyle, 2-butinyle et 3-butinyle,
un radical choisi parmi le groupe constitué de cyclopropyle, cyclobutyle, cyclopentyle, cyclohexyle, cycloheptyle, cyclo-octyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, sachant que le radical peut être substitué respectivement par 1 ou 2 substituants choisis indépendamment les uns des autres parmi le groupe constitué de -C(=O)-O-CH₃, -C(=O)-O-C₂H₅, -C(=O)-O-C(CH₃)₃, -C(=O)-CH₃, -C(=O)-C₂H₅, -C(=O)-C(CH₃)₃, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, pyridinyle, phényle et benzyle, sachant que respectivement la partie cyclique des radicaux pyridinyle, -S(=O)₂-phényle, phényle et benzyle peut être substituée par 1, 2, 3, 4 ou 5 substituants choisis indépendamment les uns des autres parmi le groupe constitué de F, Cl, Br, méthyle, éthyle, n-propyle, isopropyle, n-butyle, sec-butyle, isobutyle, tert-butyle, -O-CH₃ et -O-C₂H₅ ;
un radical choisi parmi le groupe constitué de phényle, naphthyle, thiophényle, furanyle, pyrrolyle, pyrazolyle, pyranyle, triazolyle, pyridinyle, imidazolyle, indolyle, iso-indolyle, benzo[b]furanyle, benzo[b]thiophényle, thiazolyle, oxazolyle, isoxazolyle, pyridazinyle, pyrazinyle et pyrimidinyle, sachant que le radical peut être substitué le cas échéant respectivement par 1, 2, 3, 4 ou 5 substituants choisis indépendamment les uns des autres parmi le groupe constitué de F, Cl, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -O-phényle, -O-benzyle et phényle,
ou représentent -(CH₂)-R⁴² ou -(CH₂)-(CH₂)-R⁴².

11. Composés selon une ou plusieurs des revendications 1 à 10, **caractérisés en ce que**
R²⁶, R²⁷, R²⁸, R³⁰, R³¹, R³², R³⁵, R³⁶, R³⁷, R³⁹ et R⁴⁰, représentent indépendamment les uns des autres, respectivement
un radical hydrogène ;
un radical choisi parmi le groupe constitué de méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthinyle, 1-propinyle, 2-propinyle, 1-butinyle, 2-butinyle et 3-butinyle ;
ou représentent un radical phényle, qui peut être substitué par 1, 2, 3, 4 ou 5 substituants choisis indépendamment les uns des autres parmi le groupe constitué de F, CI, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -o-phényle, -O-benzyle et phényle.

12. Composés selon une ou plusieurs des revendications 1 à 11, **caractérisés en ce que**
R²⁹, R³³ et R⁴¹ représentent, indépendamment les uns des autres, respectivement un radical choisi parmi le groupe constitué de méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthinyle, 1-propinyle, 2-propinyle, 1-butinyle, 2-butinyle et 3-butinyle, sachant que le radical peut être substitué le cas échéant respectivement par 1, 2, 3, 4 ou 5 substituants choisis indépendamment les uns des autres parmi le groupe constitué de F, Cl, Br, -CN, -NO₂, -OH, -SH et -NH₂;
un radical choisi parmi le groupe constitué de cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indanyle, (1, 4)-benzodioxanyle, (1, 2, 3, 4)-tétrahydronaphthyle, (1, 2, 3, 4)-tétrahydroquinolinyle et (1, 2, 3, 4)-tétrahydroquinazolinyle, sachant que le radical peut être substitué le cas échéant respectivement par 1, 2, 3, 4 ou 5 substituants choisis indépendamment les uns des autres parmi le groupe constitué de oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, .S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -OC(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-Butyle, Isobutyle, tert-butyle, -O-CH₃, -O-C₂H₅, -O-CF₃, -S-CF₃, phényle et -O-benzyle ;
un radical choisi parmi le groupe constitué de phényle, naphthyle, (1, 3)-benzodioxolyle, (1, 4)-benzodioxanyle, thiophényle, furanyle, pyrrolyle, pyrazolyle, pyranyle, triazolyle, pyridinyle, imidazolyle, indolyle, iso-indolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle, isoquinolinyle, benzimidazolinyle, benzoxazolyle, benzisoxazolyle et benzothiazolyle, sachant que le radical peut être substitué le cas échéant respectivement par 1, 2, 3, 4 ou 5 substituants choisis indépendamment les uns des autres parmi le groupe constitué de F, CI, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₅, -S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -O-phényle, -O-benzyle, phényle et benzyle, sachant que respectivement la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furanyle et benzyle peut être substituée par 1, 2, 3, 4 ou 5 substituants choisis indépendamment les uns des autres parmi le groupe constitué de F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle.

13. Composés selon une ou plusieurs des revendications 1 à 12, **caractérisés en ce que**
R³⁴ et R⁴², représentent respectivement, indépendamment l'un de l'autre, un radical choisi parmi le groupe constitué de phényle, naphthyle, thiophényle, furanyle, pyrrolyle, pyrazolyle, pyranyle, triazolyle, pyridinyle, imidazolyle, indolyle, iso-indolyle, benzo[b]furanyle, benzo[b]thiophényle, thiazolyle, oxazolyle, isoxazolyle, pyridazinyle, pyrazinyle et pyrimidinyle, sachant que le radical peut être substitué le cas échéant respectivement par 1, 2, 3, 4 ou 5 substituants choisis indépendamment les uns des autres parmi le groupe constitué de F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NE-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃.

14. Composés selon une ou plusieurs des revendications 1 à 13, **caractérisés en ce que**
m est égal à 0.

15. Composés selon une ou plusieurs des revendications 1 à 14, **caractérisés en ce que** les composés présentent dans l'essai FLIPR à une concentration de 10 µM une inhibition de l'afflux d'ions Ca²⁺ dans les ganglions de la racine postérieure des nerfs spinaux de rats d'au moins 10 %, de préférence d'au moins 30 %, de façon particulièrement préférée d'au moins 50 %, de façon tout particulièrement préférée d'au moins 70 %, encore plus préférablement d'au moins 90 %, en comparaison de l'inhibition maximale de l'afflux d'ions Ca²⁺ réalisable avec la capsaïcine à une concentration de 10 µM.

16. Procédé de préparation de composés spiro substitués de la formule générale 1 selon une ou plusieurs des revendications 1 à 15, **caractérisé en ce qu'**au moins un composé de formule générale II, 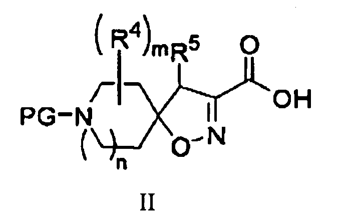 dans laquelle R⁴, R⁵, m et n ont la signification selon une ou plusieurs des revendications 1 à 15 et PG signifie un groupe de protection, de préférence un groupe benzyloxycarbonyle ou tert-butyloxycarbonyle, est mis en réaction dans un milieu réactionnel en présence d'au moins d'un réactif d'accouplement, le cas échéant en présence d'au moins une base, avec un composé de la formule générale HNR²R³, dans laquelle R² et R³ ont la signification selon une ou plusieurs des revendications 1 à 15, pour donner au moins un composé de formule générale III, 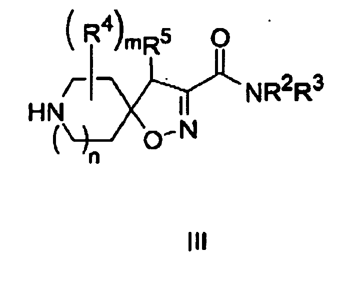 dans laquelle R², R³, R⁴, R⁵, m, n et PG ont la signification spécifiée ci-avant et celui-ci, le cas échéant, est purifié et/ou isolé
et
au moins un composé de la formule générale III est mis en réaction dans un milieu réactionnel en présence d'au moins un acide, de préférence en présence d'au moins un acide inorganique ou organique, choisi parmi le groupe constitué de l'acide chlorhydrique, de l'acide sulfurique, de l'acide acétique et de l'acide trifluoracétique, ou en présence d'hydrogène et d'un catalyseur, de préférence d'un catalyseur à base de palladium ou de platine, pour donner au moins un composé de la formule général IV, 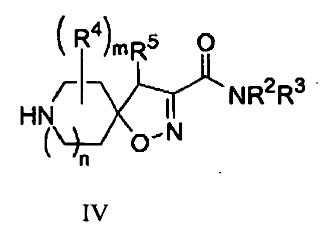 dans laquelle R², R³, R⁴, R⁵, m et n ont la signification spécifiée ci-avant, et celui-ci, le cas échéant, est purifié et/ou isolé
et
au moins un composé de la formule générale IV est mis en réaction dans un milieu réactionnel avec au moins un isocyanate de la formule générale R⁶-N=C=O, dans laquelle R⁶ a la signification selon une ou plusieurs des revendications 1 à 15, le cas échéant en présence d'au moins une base, de préférence en présence d'au moins une base choisie parmi le groupe constitué de la triéthylamine, de la 4,4-diméthylaminopyridine et de la diisopropyléthylamine, pour donner au moins un composé de la formule général I, dans laquelle R², R³, R⁴, m et n ont la signification spécifiée ci-avant et R¹ représente -C(=O)-NR⁶R⁷, sachant que R⁶ a la signification spécifiée ci-avant et R⁷ représente un radical hydrogène, et ce composé est le cas échéant purifié et/ou isolé
ou
au moins un composé de la formule générale IV est mis en réaction dans un milieu réactionnel avec au moins un isothiocyanate de la formule générale S=C=N-R⁸, dans laquelle R⁸ a la signification selon une ou plusieurs des revendications 1 à 15, le cas échéant en présence d'au moins une base, de préférence en présence d'au moins une base choisie parmi le groupe constitué de la 4, 4-diméthylaminopyridine et de la diisopropyléthylamine, pour donner au moins un composé de la formule générale I, dans laquelle R², R³, R⁴, R⁵, m et n ont la signification spécifiée ci-avant et R¹ représente -C(=S)-N-R⁸R⁹, sachant que R⁸ a la signification spécifiée ci-avant et R⁹ représente un radical hydrogène, et ce composé est le cas échéant purifié et/ou isolé
et le cas échéant au moins un composé de la formule générale I, dans laquelle R², R³, R⁴, R⁵, m et n ont la signification spécifiée ci-avant et R¹ représente -C(=O)-NR⁶R⁷ ou -C(=S)-N-R⁸R⁹, dans laquelle formule R⁷ et R⁹ représentent chacun un radical hydrogène, sont mis en réaction dans un milieu réactionnel, en présence d'au moins une base, de préférence en présence d'au moins un sel d'hydrure métallique ou un sel d'alcoolate métallique, de façon particulièrement préférée en présence d'un sel d'hydrure métallique ou d'un sel d'alcoolate métallique choisi parmi le groupe constitué de l'hydrure de sodium, de l'hydrure de potassium, du tert-butanolate de potassium, du tert-butanolate de sodium, du méthanolate de potassium, du méthanolate de sodium, de l'éthanolate de sodium et de l'éthanolate de potassium, avec au moins un composé de la formule générale LG-R⁷ ou de la formule générale LG-R⁹, dans laquelle LG représente un groupe partant, de préférence un atome d'halogène, de façon particulièrement préférée un atome de chlore, et R⁷ et R⁹ ont la signification spécifiée ci-avant à l'exception de l'hydrogène, pour donner au moins un composé de la formule générale I, dans laquelle R¹ à R⁵, m et n ont la signification spécifiée ci-avant, et ce composé est le cas échéant purifié et/ou isolé,
ou
au moins un composé de la formule générale IV est mis en réaction dans un milieu réactionnel, en présence d'au moins une base, de préférence en présence d'au moins un sel d'hydrure métallique, de façon particulièrement préférée en présence d'hydrure de sodium et/ou d'hydrure de potassium, avec au moins un composé de la formule générale LG-R¹, dans laquelle R¹ a la signification selon une ou plusieurs des revendications 1 à 15, à l'exception de -C(=O)-NR⁶R⁷ et de -C(=S)-NR⁸R⁹, et LG représente un groupe partant, de préférence un atome d'halogène, de façon particulièrement préférée un atome de chlore, pour donner au moins un composé de la formule générale I, dans laquelle R¹ à R⁵, m et n ont la signification spécifiée ci-avant, et ce composé est le cas échéant purifié et/ou isolé
ou
au moins un composé de la formule générale IV est mis en réaction dans un milieu réactionnel, en présence d'au moins un agent réducteur, avec au moins un composé de la formule générale R¹-C(=O)-H, dans laquelle R¹ a la signification selon une ou plusieurs des revendications 1 à 15, à l'exception de -C(=O)-NR⁶R⁷ et -C(=S)-NR⁸R⁹, pour donner au moins un composé de la formule générale I, dans laquelle R¹ à R⁵, m et n ont la signification spécifiée ci-avant, et ce composé est le cas échéant purifié et/ou isolé.

17. Produit pharmaceutique contenant au moins un composé selon une ou plusieurs des revendications 1 à 15 et le cas échéant une ou plusieurs substances auxiliaires physiologiquement compatibles.

18. Produit pharmaceutique selon la revendication 17 pour la prophylaxie et/ou le traitement de douleurs, de préférence de douleurs choisies parmi le groupe constitué de la douleur aiguë, de la douleur chronique, de la douleur névropathique et de la douleur viscérale.

19. Produit pharmaceutique selon la revendication 17 pour le traitement et/ou la prophylaxie d'une ou de plusieurs affections, choisies parmi le groupe constitué des douleurs, de préférence des douleurs choisies parmi le groupe constitué de la douleur aiguë, de la douleur chronique, de la douleur névropathique, de la douleur viscérale ; de la douleur articulaire ; de la migraine ; des dépressions ; des névropathies ; des traumatismes nerveux ; des affections neurodégéneratives, choisies de préférence parmi le groupe constitué de la sclérose en plaques, de la maladie d'Alzheimer, de la maladie de Parkinson et de la maladie d'Huntington ; des dysfonctions cognitives, de préférence des états de carence cognitive, de façon particulièrement préférée des troubles de la mémoire ; de l'épilepsie ; des affections des voies respiratoires, choisies de préférence parmi le groupe constitué de l'asthme et de la pneumonie ; de la toux ; de l'incontinence urinaire ; d'une hyperactivité de la vessie (overactive bladder, OAB) ; d'ulcères à l'estomac ; du syndrome d'irritabilité des intestins ; de l'apoplexie ; des irritations oculaires ; des irritations cutanée ; des névrodermites ; des maladies inflammatoires, de préférence des inflammations de l'intestin ; de la diarrhée ; du prurit ; des perturbations de la prise d'aliments, choisies de préférence parmi le groupe constitué de la boulimie, de la cachexie, de l'anorexie et de l'obésité ; de l'addiction médicamenteuse ; de l'abus de médicaments ; des syndromes de sevrage en cas d'addiction médicamenteuse ; du développement de tolérance vis-à-vis de médicaments, de préférence vis-à-vis des opioïdes naturels ou synthétiques ; de la toxicomanie ; de l'abus de drogues ; des syndromes de sevrage en cas de toxicomanie ; de l'addiction alcoolique ; de l'alcoolisme et des syndromes de sevrage en cas d'alcoolisme ; pour la diurèse ; pour l'antinatriurèse ; pour l'influencement du système cardiovasculaire ; pour l'accroissement de la vigilance ; pour l'accroissement de la libido ; pour la modulation de l'activité de mouvement ; pour l'anxiolyse ; pour l'anesthésie locale et/ou pour l'inhibition d'effets secondaires indésirables, choisis de préférence parmi le groupe constitué de l'hyperthermie, de l'hypertension artérielle et du rétrécissement des bronches, déclenché par l'administration d'agonistes de récepteurs de vanilloïde 1 (récepteurs VR1/TRPV1), choisis de préférence parmi le groupe constitué de la capsaïcine, de la résinifératoxine, de l'olvanil, de l'arvanil, du SDZ-249665, du SDZ-249482, du nuvanil et du capsavanil (DA-5018).

20. Utilisation d'au moins un composé selon une ou plusieurs des revendications 1 à 15 pour la préparation d'un produit pharmaceutique pour la prophylaxie et/ou le traitement de douleurs, de préférence de douleurs choisies parmi le groupe constitué de la douleur aiguë, de la douleur chronique et de la douleur névropathique.

21. Utilisation d'au moins un composé selon une ou plusieurs des revendications 1 à 15 pour la préparation d'un produit pharmaceutique pour la prophylaxie et/ou le traitement d'une ou de plusieurs affections choisies parmi le groupe constitué des douleurs, de préférence des douleurs choisies parmi le groupe constitué de la douleur aiguë, de la douleur chronique, de la douleur névropathique, de la douleur viscérale ; de la douleur articulaire ; de la migraine ; des dépressions ; des névropathies ; des traumatismes nerveux ; des affections neurodégéneratives, choisies de préférence parmi le groupe constitué de la sclérose en plaques, de la maladie d'Alzheimer, de la maladie de Parkinson et de la maladie d'Huntington ; des dysfonctions cognitives, de préférence des états de carence cognitive, de façon particulièrement préférée des troubles de la mémoire ; de l'épilepsie ; des affections des voies respiratoires, choisies de préférence parmi le groupe constitué de l'asthme et de la pneumonie ; de la toux ; de l'incontinence urinaire ; d'un hyperactivité de la vessie (overactive bladder, OAB) ; d'ulcères à l'estomac ; du syndrome d'irritabilité des intestins ; de l'apoplexie ; des irritations oculaires ; des irritations cutanée ; des névrodermites ; des maladies inflammatoires, de préférence des inflammations de l'intestin ; de la diarrhée ; du prurit ; des perturbations de la prise d'aliments, choisies de préférence parmi le groupe constitué de la boulimie, de la cachexie, de l'anorexie et de l'obésité ; de l'addiction médicamenteuse ; de l'abus de médicaments ; des syndromes de sevrage en cas d'addiction médicamenteuse ; du développement de tolérance vis-à-vis de médicaments, de préférence vis-à-vis des opioïdes naturels ou synthétiques ; de la toxicomanie ; de l'abus de drogues ; des syndromes de sevrage en cas de toxicomanie ; de l'addiction alcoolique ; de l'alcoolisme et des syndromes de sevrage en cas d'alcoolisme ; pour la diurèse ; pour l'antinatriurèse ; pour l'influencement du système cardiovasculaire ; pour l'accroissement de la vigilance ; pour l'accroissement de la libido ; pour la modulation de l'activité de mouvement ; pour l'anxiolyse ; pour l'anesthésie locale et/ou pour l'inhibition d'effets secondaires indésirables, choisis de préférence parmi le groupe constitué de l'hyperthermie, de l'hypertension artérielle et du rétrécissement des bronches, déclenché par l'administration d'agonistes de récepteurs de vanilloïde 1 (récepteurs VR1/TRPV1), choisis de préférence parmi le groupe constitué de la capsaïcine, de la résinifératoxine, de l'olvanil, de l'arvanil, du SDZ-249665, du SDZ-249482, du nuvanil et du capsavanil (DA-5018).
